# EUROPEAN PATENT SPECIFICATION

(11) **EP 2 792 665 B1**
(45) Date of publication and mention of the grant of the patent: **03.08.2016**
(21) Application number: 12857476.1
(22) Date of filing: 13.12.2012
(51) Int. Cl.: C07C 69/708, C08F 220/30, C09D 4/02, C09D 157/08

(54) **LIQUID REPELLENT COMPOSITION, LIQUID REPELLENT POLYMER, CURABLE COMPOSITION, COATING COMPOSITION, ARTICLE HAVING CURED FILM, ARTICLE HAVING PATTERN OF LIQUID-PHILIC REGION AND LIQUID REPELLENT REGION, AND PROCESS FOR PRODUCING IT**
WASSERABWEISENDE ZUSAMMENSETZUNG, WASSERABWEISENDES POLYMER, HÄRTBARE ZUSAMMENSETZUNG, BESCHICHTUNGSZUSAMMENSETZUNG, ARTIKEL MIT EINEM GEHÄRTETEN FILM, ARTIKEL MIT EINEM MUSTER MIT EINER LYOPHILEN UND EINER WASSERABWEISENDEN REGION SOWIE HERSTELLUNGSVERFAHREN DAFÜR
COMPOSITION REPOUSSANT LES LIQUIDES, POLYMÈRE REPOUSSANT LES LIQUIDES, COMPOSITION DURCISSABLE, COMPOSITION DE REVÊTEMENT, ARTICLE PORTANT UN FILM DURCI, ARTICLE PORTANT UN MOTIF CONSTITUÉ D'UNE RÉGION LIQUIPHILE ET D'UNE RÉGION REPOUSSANT LES LIQUIDES, ET LEUR PROCÉDÉ DE PRODUCTION

(30) Priority: 15.12.2011 JP 2011274320
(43) Date of publication of application: 22.10.2014
(73) Proprietor: Asahi Glass Company, Limited, Tokyo 100-8405 (JP)
(72) Inventor: ITO, Masahiro, Tokyo 100-8405 (JP); TSURUOKA, Kaori, Tokyo 100-8405 (JP)
(74) Representative: Müller-Boré & Partner Patentanwälte PartG mbB
(86) International application number: PCT/JP2012/082424
(87) International publication number: WO 2013/089204

(56) References cited:
- JP-A- H02 188 561
- JP-A- H05 127 394
- JP-A- S61 118 467
- JP-A- 2009 242 679
- JP-A- 2010 196 044
- JP-A- 2010 529 260
- JP-A- 2011 008 001
- US-A1- 2009 047 602

## Description

### TECHNICAL FIELD

The present invention relates to a liquid repellent composition which can be converted to be liquid-philic by irradiation with ultraviolet light, a liquid repellent polymer, a curable composition, a coating composition, an article having a cured film, an article having a pattern of a liquid-philic region and a liquid repellent region, and a process for producing it.

### BACKGROUND ART

In the field of semiconductor devices, displays, light-emitting devices, etc., various functional thin-films are practically used. A functional thin film is patterned by disposing a material having desired properties to a desired position. For example, for a thin-film transistor, a process for producing an electrode has been proposed by which a cured film having liquid repellency is formed on the surface of a substrate, the surface of the cured film is partially irradiated with ultraviolet light to convert the portion irradiated with ultraviolet light to be liquid-philic, and a composition for an electrode is selectively deposited to the portion of the cured film converted to be liquid-philic to form an electrode. This method attracts attention as a method to form an electrode having a desired pattern easily with a small number of steps as compared with a method by photolithography.

As a material for forming a liquid repellent cured film which can be converted to be liquid-philic by irradiation with ultraviolet light, for example, the following materials have been proposed.
(1) A material containing a photocatalyst (such as titanium dioxide) and a binder (such as organopolysiloxane) (Patent Document 1).
(2) A composition containing a low molecular weight fluorinated compound which can be decomposed and removed by irradiation with ultraviolet light (Patent Document 2).
(3) A polyimide having hydrophobic groups in its side chains (Patent Document 3).
(4) A polyimide having a thiolester bond in its main chain (Patent Document 4).

US 2009/047602 A1 discloses monomers for the preparation of a water-repellent resist film and containing acrylate and fluorinated moieties separated by a linker - [(OCH₂CH₂)ₙ]ₘ-X-C₆H₄-CO-CR₁R₂-O-CO-.

### PRIOR ART DOCUMENTS

### PATENT DOCUMENTS

Patent Document 1: Japanese Patent No. 4300012
Patent Document 2: WO2005/054256
Patent Document 3: JP-A-2005-310962
Patent Document 4: WO2008/133300

### DISCLOSURE OF INVENTION

### TECHNICAL PROBLEM

In a case where a liquid repellent cured film is formed by using the above material (1), the photocatalyst remains in the cured film. In a case where an article having a functional thin-film is a thin-film transistor, a semiconductor device or the like, the cured film functions as an insulation film, and the photocatalyst remaining in the insulation film adversely effects the properties (such as the insulating property) of the insulation film.

Further, in a case where a liquid repellent cured film is formed by using the above composition (2) containing a fluorinated compound, since the fluorinated compound has a relatively low molecular weight, the fluorinated compound is uniformly dispersed in the cured film, and the fluorinated compound will not be unevenly present on the surface of the cured film (the opposite side from the substrate). Accordingly, the liquid repellency of the surface of the cured film may sometimes be insufficient. Further, the obtained cured film may sometimes have insufficient insulating properties.

Further, in a case where a liquid repellent cured film is formed by using the above polyimide (3) or (4), the polyimide absorbs ultraviolet light at a relatively long wavelength side (for example, ultraviolet light having a wavelength of at least 300 nm). Accordingly, in a case where a light source commonly used as a light source for ultraviolet light such as a high pressure mercury lamp (i-line: 365 nm) or a YAG laser (third harmonic: 355 nm) is used, the sensitivity tends to be insufficient. Further, in recent years, a material having a dielectric constant lower than that of the polyimide is required in some cases.

The object of the present invention is to provide a liquid repellent compound which has favorable liquid repellency, which is decomposed in its molecule by irradiation with ultraviolet light having a wavelength of at least 300 nm, and from which a decomposition residue containing a Cf group can leave, a liquid repellent polymer, and a curable composition and a coating composition, from which a liquid repellent cured film having favorable insulating property and liquid repellency and sufficiently converted to be liquid-philic by irradiation with ultraviolet light having a wavelength of 300 nm, can be formed.

Another object of the present invention is to provide an article having a cured film having favorable insulating property and liquid repellency, an article having favorable insulating property and having a pattern of a liquid-philic region and a liquid repellent region, and its production process.

### SOLUTION TO PROBLEM

The present invention provides a liquid repellent compound, a liquid repellent polymer, a curable composition, a coating composition, an article having a cured film, an article having a pattern of a liquid-philic region and a liquid repellent region, and its production process according to the following [1] to [15].
[1] A liquid repellent compound represented by the following formula (m1): 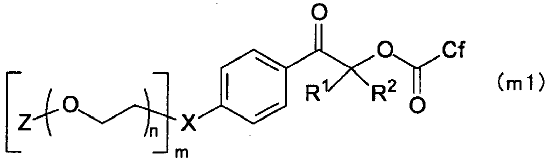 wherein Cf is a C₁₋₂₀ fluoroalkyl group or a C₁₋₂₀ fluoroalkyl group having an etheric oxygen atom between carbon atoms,
   each of R¹ and R² which are independent of each other, is a hydrogen atom, a C₁₋₆ alkyl group or a phenyl group,
   X is an oxygen atom, a sulfur atom, a nitrogen atom or NH, n in an integer of from 0 to 4,
   m is 1 when X is an oxygen atom, a sulfur atom or NH, or 2 when X is a nitrogen atom,
   Z is R⁴R⁵C=CR³-CO-, and
   each of R³, R⁴ and R⁵ which are independent of one another, is a hydrogen atom or a methyl group.
[2] A liquid repellent polymer having units (u1) based on the liquid repellent compound as defined in the above [1].
[3] The liquid repellent polymer according to the above [2], which further has units (u2) having a crosslinkable functional group and having no Cf group.
[4] A curable composition comprising the liquid repellent polymer as defined in the above [2] or [3].
[5] The curable composition according to the above [4], which further contains a radical polymerization initiator (D).
[6] The curable composition according to the above [4] or [5], which further contains a fluorinated polyarylene prepolymer (A) having a crosslinkable functional group.
[7] The curable composition according to the above [6], which further contains a compound (B) having a number average molecular weight of from 140 to 5,000, having a crosslinkable functional group and having no fluorine atoms.
[8] The curable composition according to any one of the above [4] to [7], wherein the fluorinated polyarylene prepolymer (A) is a prepolymer having a crosslinkable functional group and an ether bond, obtained by subjecting either one or both of a compound (x1) having a crosslinkable functional group and a phenolic hydroxy group and a compound (x2) having a crosslinkable functional group and a fluorinated aromatic ring, a compound (y) represented by the following formula (y): 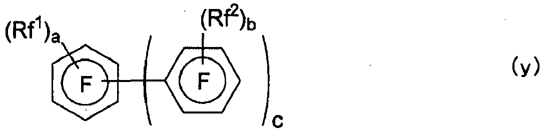 (wherein a is an integer of from 0 to 3, b is an integer of from 0 to 3, c is an integer of from 0 to 3, each of Rf¹ and Rf² which are independent of each other, is a fluoroalkyl group having at most 8 carbon atoms, and F in the aromatic ring represents that hydrogen atoms of the aromatic ring are all substituted by fluorine atoms), and a compound (z) having at least three phenolic hydroxy groups, to a condensation reaction in the presence of a hydrogen halide-removing agent.
[9] A coating composition comprising the curable composition as defined in any one of the above [4] to [8] and a solvent (E).
[10] An article comprising a substrate, and a cured film obtained by curing the curable composition as defined in any one of the above [4] to [8] on the surface of the substrate.
[11] An article having a pattern of a liquid-philic region and a liquid repellent region on the surface of a cured film, wherein the liquid repellent region comprises a cured film obtained by curing the curable composition as defined in any one of the above [4] to [8].
[12] A process for producing an article having a pattern of a liquid-philic region and a liquid repellent region on the surface of a cured film, which comprises the following steps (I) and (II):
   (I) a step of applying the coating composition as defined in the above [9] on the surface of a substrate, and removing the solvent (E), followed by heating or light irradiation to form a cured film, and
   (II) a step of partially irradiating the surface of the cured film with ultraviolet light to obtain an article having a pattern of a liquid-philic region and a liquid repellent region on the surface of the cured film.
[13] The process for producing an article according to the above [12], which further has the following step (III) after the step (II):
   (III) a step of selectively depositing at least one member selected from the group consisting of a composition for an electrode, a composition for a semiconductor layer, a composition for a conductor layer, a composition for a transistor material and a composition for a resin layer to the surface of the liquid-philic region to form at least one member selected from the group consisting of an electrode, a semiconductor layer, a conductor layer, a transistor material and a resin layer.
[14] The article according to the above [10], which further has at least one member selected from the group consisting of an electrode, a semiconductor layer, a conductor layer, a transistor material and a resin layer, formed on the surface of the liquid-philic region.
[15] The article according to the above [14], which is a thin-film transistor.

### ADVANTAGEOUS EFFECTS OF INVENTION

According to the present invention, it is possible to provide a liquid repellent compound and a liquid repellent polymer, which have favorable liquid repellency, which are decomposed in their molecule by irradiation with ultraviolet light having a wavelength of at least 300 nm, and from which a decomposition residue containing a Cf group can leave, and a curable composition and a coating composition, from which a liquid repellent cured film having favorable insulating property and liquid repellency, sufficiently converted to be liquid-philic by irradiation with ultraviolet light having a wavelength of at least 300 nm, can be formed.

Further, according to the present invention, it is possible to provide an article having a cured film having favorable insulating property and liquid repellency, an article having favorable insulating property and having a pattern of a liquid-philic region and a liquid repellent region, and a process for efficiently producing the article.

### BRIEF DESCRIPTION OF DRAWINGS

Fig. 1 is a cross-sectional view illustrating an example of an organic thin-film transistor.
Fig. 2 is a cross-sectional view illustrating an example of a step for producing an organic thin-film transistor.
Fig. 3 is a cross-sectional view illustrating an example of a step for producing an organic thin-film transistor.

### DESCRIPTION OF EMBODIMENTS

In this specification, a compound represented by a formula (m1) will be referred to as a compound (m1). The same applies to compounds represented by other formulae.

In this specification, "liquid repellency" generally means water repellency and oil repellency.

In this specification, "liquid-philicity" generally means hydrophilicity and lipophilicity. In this specification, "converted to be liquid-philic" means that liquid-repellency is relatively changed to liquid-philicity, and specifically, the contact angle to water or an organic solvent is decreased.

In this specification, "fluoroalkyl group" means an alkyl group in which some or all of the hydrogen atoms are substituted by fluorine atoms, and "perfluoroalkyl group" is an alkyl group in which all the hydrogen atoms are substituted by fluorine atoms.

In this specification, "methacryloyl(oxy) group" generally means a methacryloyl group and a methacryloyloxy group. The same applies to "acryloyl(oxy) group".

In this specification, "(meth)acryloyl group" generally means an acryloyl group and a methacryloyl group. The same applies to "(meth)acryloyloxy group".

In this specification, "unit" is a unit derived from a monomer, formed by polymerization of the monomer. The unit may be a unit directly formed by polymerization, or may be a unit having a part of the unit converted to another structure by treatment of the polymer.

In this specification, "monomer" means a compound having a functional group polymerizable by radicals.

In this specification, "crosslinkable functional group" means a functional group polymerizable by radicals.

The crosslinkable functional group may, for example, be a carbon-carbon unsaturated double bond which is polymerizable by radicals, a carbon-carbon unsaturated triple bond which is polymerizable by radicals, a ring which is to be opened by radicals, and groups containing them. The unsaturated double bond and the unsaturated triple bond may be present at the inside of a molecular chain (hereinafter, also referred to as "inside olefin type") or present at a terminal thereof (hereinafter, also referred to as "terminal olefin type"), but a terminal olefin type is preferred since its reactivity is high. The inside olefin type includes a case where an unsaturated double bond is present in a part of an aliphatic ring, such as cycloolefin. The terminal olefin type crosslinkable functional group is preferably an alkenyl group having at most 4 carbon atoms or an alkynyl group having at most 4 carbon atoms.

Specifically, the crosslinkable functional group may be a vinyl group, an allyl group, an isopropenyl group, a 3-butenyl group, a methacryloyl group, a methacryloyloxy group, an acryloyl group, an acryloyloxy group, a vinyloxy group, an allyloxy group, a trifluorovinyl group, a trifluorovinyloxy group, an ethynyl group, a 1-oxocyclopenta-2,5-dien-3-yl group, a cyano group, an alkoxysilyl group, a diarylhydroxymethyl group, a hydroxyfluorenyl group, a cyclobutalene ring or an oxirane ring.

The crosslinkable functional group is preferably at least one member selected from the group consisting of a vinyl group, an allyl group, an ethynyl group, a vinyloxy group, an allyloxy group, an acryloyl group, an acryloyloxy group, a methacryloyl group and a methacryloyloxy group, since the reactivity is high, and a cured film having high crosslinking density can easily be obtained.

In this specification, the number average molecular weight (Mn) is a molecular weight calculated as polystyrene obtainable by measurement by gel permeation chromatography employing a calibration curve prepared by using a standard polystyrene sample having a known molecular weight.

### [Liquid repellent compound]

The liquid repellent compound in the present invention is a compound (m1):

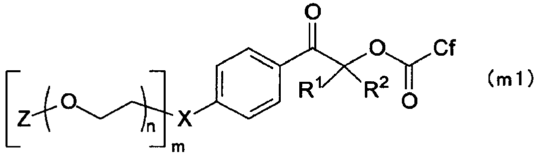

Cf is a C₁₋₂₀ fluoroalkyl group or a C₁₋₂₀ fluoroalkyl group having an etheric oxygen atom between carbon atoms.

The Cf group has preferably from 2 to 20 carbon atoms, more preferably from 2 to 15, particularly preferably from 4 to 8 carbon atoms, in view of excellent liquid repellency and favorable compatibility with another monomer. Further, the Cf group preferably has at most 6, more preferably from 2 to 6, particularly preferably from 4 to 6 carbon atoms, in view of the low environmental burden.

The Cf group has a proportion of the number of fluorine atoms of at least 80% to the total number of fluorine atoms and hydrogen atoms, in view of more favorable liquid repellency of the surface of a cured film, particularly preferably 100%, that is, the Cf group is a C₁₋₂₀ perfluoroalkyl group or a C₁₋₂₀ perfluoroalkyl group having an etheric oxygen atom between carbon atoms.

The Cf group may be linear or branched.

Specifically, the Cf group may be -CF₃, -CF₂CF₃, -CF(CF₃)₂, -CH(CF₃)₂, -CF₂CHF₂, -(CF₂)₂CF₃, -(CF₂)₃CF₃, -(CF₂)₄CF₃, -(CF₂)₅CF₃, -(CF₂)₆CF₃, -(CF₂)₇CF₃, -(CF₂)₈CF₃, -(CF₂)₉CF₃, -(CF₂)₁₁CF₃, -(CF₂)₁₅CF₃, -CF(CF₃)O(CF₂)₅CF₃, -CF₂OCF₂CF₂OCF₂CF₃ -CF₂O(CF₂CF₂O)ₚCF₃ (wherein p is an integer of from 1 to 8), -CF(CF₃)O(CF₂CF(CF₃)O)_{q}C₆F₁₃ (wherein q is an integer of from 1 to 4), or -CF(CF₃)O(CF₂CF(CF₃)O)ᵣC₃F₇ (wherein r is an integer of from 0 to 5).

Each of R¹ and R² which are independent of each other, is a hydrogen atom, a C₁₋₆ alkyl group or a phenyl group, it is preferred that at least one of R¹ and R² is not a hydrogen atom, it is more preferred that both of R¹ and R² are not a hydrogen atom, and it is particularly preferred that both of R¹ and R² are a methyl group, whereby a decomposition residue containing the Cf group is likely to leave by irradiation with ultraviolet light.

X is an oxygen atom, a sulfur atom, a nitrogen atom or NH, preferably an oxygen atom, a sulfur atom or NH, whereby a liquid repellent polymer (C) is easily produced (the liquid repellent polymer (C) is less likely to be gelated), and is particularly preferably an oxygen atom, in view of availability of the raw material.

m is 1 when X is an oxygen atom, a sulfur atom or NH, or 2 when X is a nitrogen atom, and is preferably 1, whereby the liquid repellent polymer (C) is easily produced (the liquid repellent polymer (C) is less likely to be gelated).

Here, the liquid repellent polymer (C) is a liquid repellent polymer having units (u1) based on the liquid repellent compound (m1).

n is an integer of from 0 to 4, preferably an integer of from 0 to 2, particularly preferably an integer of from 0 to 1 in view of availability of the raw material and easiness of preparation.

Z is R⁴R⁵C=CR³-CO-.

Each of R³, R⁴ and R⁵ which are independent of one another, is a hydrogen atom or a methyl group, preferably a hydrogen atom in view of high reactivity. That is, Z is preferably an acryloyl group.

### (Method for producing compound (m1))

To produce the compound (m1), a method to carry out reaction represented by the following formula may be mentioned. HO- on the left side of a compound (a1) is selectively esterified with a compound (b1) in the presence of a tertiary amine to obtain a compound (c1), and -OH on the right side of the compound (c1) is esterified with a compound (d1) to obtain a compound (m1):

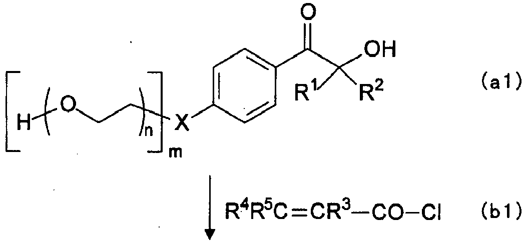

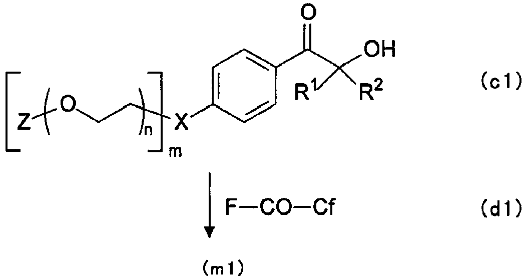

The compound (a1) may be produced, in the case of a compound wherein each of R¹ and R² is a methyl group, X is an oxygen atom and n is 1, by a process for producing "4-(2-hydroxyethoxy)phenyl 2-hydroxy-2-propylketone" disclosed in JP-A-62-81345. As a commercial product, IRGACURE 2959 (tradename, manufactured by Ciba Specialty Chemicals) may, for example, be mentioned.

### [Liquid repellent polymer]

The liquid repellent polymer of the present invention is a liquid repellent polymer (hereinafter sometimes referred to as "liquid repellent polymer (C)") having units (hereinafter sometimes referred to as "units (u1)" based on the liquid repellent compound of the present invention.

The liquid repellent polymer (C) preferably further has units (hereinafter sometimes referred to as "units (u2)") having a crosslinkable functional group and having no Cf group in view of the hardness, the solvent resistance, etc. of a water repellent film.

The liquid repellent polymer (C) may have other units (u3) other than the units (u1) and the units (u2).

The units (u1), the units (u2) and other units (u3) in the liquid repellent polymer (C) may be bonded randomly or may be bonded in a block.

The fluorine content of the liquid repellent polymer (C) is preferably from 5 to 70 mass%, more preferably from 5 to 60 mass%, particularly preferably from 8 to 60 mass%. When the fluorine content is at least the lower limit value of the above range, the liquid repellency of the surface of a cured film will be more favorable. When it is at most the upper limit value of the above range, the adhesion between the cured film and a layer adjacent thereto will be favorable.

The number average molecular weight (Mn) of the liquid repellent polymer (C) is preferably from 1,000 to 50,000, particularly preferably from 3,000 to 20,000. When the number average molecular weight (Mn) is at least the lower limit value of the above range, the liquid repellent polymer (C) will sufficiently moves to the surface of the cured film, whereby favorable liquid repellency will be obtained. When it is at most the upper limit value of the above range, compatibility with the prepolymer (A) in the curable composition will be favorable, and a cured film without defects will be formed.

### (Units (u1))

The unit (u1) is a unit derived from the compound (m1) formed by polymerization of the compound (m1). The carbon-carbon unsaturated double bond in the Z group (the group of the same kind as the crosslinkable functional group) in the compound (m1) is lost by polymerization, and accordingly the unit (u1) has no crosslinkable functional group.

The proportion of the units (u1) in the liquid repellent polymer (C) is preferably from 10 to 90 mass%, more preferably from 15 to 90 mass%, further preferably from 20 to 90 mass%, particularly preferably from 30 to 90 mass%. When the proportion of the units (u1) is at least the lower limit value of the above range, the liquid repellency of the surface of the cured film will be more favorable. When it is at most the upper limit value of the above range, the liquid repellent polymer (C) is easily soluble in a solvent (E) for the coating composition.

### (Unit (u2))

The unit (u2) is a unit having a crosslinkable functional group and having no Cf group.

The crosslinkable functional group in the unit (u2) reacts with a crosslinkable functional group of the after-mentioned fluorinated polyarylene prepolymer (hereinafter referred to as "prepolymer (A)") or the compound (B), and they are integrated to form a cured film having high hardness and excellent solvent resistance.

The number of the crosslinkable functional group in the unit (u2) is preferably 1 in view of availability of the raw material and easiness of preparation.

The crosslinkable functional group in the unit (u2) is preferably a (meth)acryloyl(oxy) group in view of high reactivity with the crosslinkable functional group of the prepolymer (A) or the compound (B).

The crosslinkable functional group in the compound (B) and the crosslinkable functional group in the liquid repellent polymer (C) which coexist in the curable composition may be the same or different from each other.

The polymerizable functional group (the group of the same kind as the crosslinkable functional group) which the monomer has is lost by polymerization, and accordingly the crosslinkable functional group of the unit (u2) is not the polymerizable functional group which the monomer originally has. Accordingly, the crosslinkable functional group of the unit (u2) is usually a crosslinkable functional group introduced afterwards e.g. by modification to a copolymer obtained by polymerization of the monomer.

The crosslinkable functional group of the unit (u2) is preferably introduced by a modification method of reacting a copolymer having reactive functional groups with a compound having a crosslinkable functional group. As the modification method, a known method may properly be used. Specifically, a compound (m4) having a polymerizable functional group and a reactive functional group is copolymerized with the compound (m1) to obtain a copolymer having units (u4) having a reactive functional group, which is reacted with a compound (a2) having a functional group reactive with the reactive functional group of the unit (u4) and a crosslinkable functional group to obtain a liquid repellent polymer (C) having units (u2). The unit (u2) is a unit formed by bonding of the unit (u4) formed by polymerization of the compound (m4) and the compound (a2).

The reactive functional group may, for example, be a hydroxy group, an epoxy group or a carboxy group. In a case where the reactive functional group is a hydroxy group, the functional group reactive with the reactive functional group may, for example, be a carboxy group, an isocyanate group or acyl chloride. In a case where the reactive functional group is an epoxy group, the functional group reactive with the reactive functional group may, for example, be a carboxy group. In a case where the reactive functional group is a carboxy group, the functional group reactive with the reactive functional group may, for example, be a hydroxy group or an epoxy group.

As a specific modification method, for example, the following methods (i) to (vi) may, for example, be mentioned.
(i) A method of reacting a copolymer obtained by copolymerizing a monomer having a hydroxy group with an acid anhydride having a crosslinkable functional group.
(ii) A method of reacting a copolymer obtained by copolymerizing a monomer having a hydroxy group with a compound having an isocyanate group and a crosslinkable functional group.
(iii) A method of reacting a copolymer obtained by copolymerizing a monomer having a hydroxy group with a compound having an acyl chloride group and a crosslinkable functional group.
(iv) A method of reacting a copolymer obtained by copolymerizing an acid anhydride having a polymerizable functional group with a compound having a hydroxy group and a crosslinkable functional group.
(v) A method of reacting a copolymer obtained by copolymerizing a monomer having a carboxy group with a compound having an epoxy group and a crosslinkable functional group.
(vi) A method of reacting a copolymer obtained by copolymerizing a monomer having an epoxy group with a compound having a carboxy group and a crosslinkable functional group.

In a case where the copolymer having units (u4) is reacted with the compound (a2), the compound (a2) may be reacted to all the reactive functional groups of the copolymer, or may be reacted to some of the reactive functional groups of the copolymer. In the latter case, the obtained liquid repellent polymer (C) has units (u4) derived from the compound (m4).

The liquid repellent polymer (C) to be used for the curable composition may have units (u4). Further, in a case where the reactive functional group of the unit (u4) may have unfavorable influences over the curable composition, with the reactive functional group of the unit (u4), a compound (b2) having a functional group reactive with the reactive functional group and having no crosslinkable functional group may be reacted to convert the reactive functional group to an inert group.

The compound (b2) may, for example, ethyl isocyanate, propyl isocyanate, isopropyl isocyanate, butyl isocyanate, t-butyl isocyanate, hexyl isocyanate, cyclohexyl isocyanate, dodecyl isocyanate, octadecyl isocyanate, phenyl isocyanate, acetyl chloride, propionyl chloride, butyryl chloride, isobutyl chloride, pivaloyl chloride, isovaleryl chloride, valeryl chloride, 3,3-dimethylbutyryl chloride, hexanoyl chloride, heptanoyl chloride, 2-ethylhexanoyl chloride, octanoyl chloride, nonanoyl chloride, decanoyl chloride or lauroyl chloride, and is preferably ethyl isocyanate, propyl isocyanate, acetyl chloride or propionyl chloride.

A unit of which the reactive functional group is converted to an inert group will be referred to as a unit (u5).

The compound (m4) may, for example, be 2-hydroxyethyl (meth)acrylate or 4-hydroxybutyl (meth)acrylate in the method (i), (ii) or (iii). In the method (iv), it may, for example, be maleic anhydride, itaconic anhydride, citraconic anhydride or phthalic anhydride. In the method (v), it may, for example, be (meth)acrylic acid. In the method (vi), it may, for example, be glycidyl (meth)acrylate or 3,4-epoxycyclohexyl methyl acrylate.

The compound (a2) may, for example, be maleic anhydride, itaconic anhydride, citraconic anhydride or phthalic anhydride in the method (i). In the method (ii), it may, for example, be 2-(meth)acryloyloxyethyl isocyanate or 1,1-bis(acryloyloxymethyl)ethylisocyanate. In the method (iii), it may, for example, be (meth)acrylateacryloyl chloride or 3-butenoyl chloride. In the method (iv), it may, for example, be 2-hydroxyethyl (meth)acrylate or 4-hydroxybutyl (meth)acrylate. In the method (v), it may, for example, be glycidyl (meth)acrylate or 3,4-epoxycyclohexyl methyl acrylate. In the method (vi), it may, for example, be (meth)acrylic acid.

The unit (u2) is preferably a unit obtained by reacting a unit derived from a monomer having a hydroxy group with a compound having an isocyanate group and a crosslinkable functional group, or a unit obtained by reacting a unit derived from a monomer having a hydroxy group with a compound having an acyl chloride group and a crosslinkable functional group. Particularly preferred is a unit formed by reacting a unit derived from at least one monomer selected from the group consisting of 2-hydroxyethyl (meth)acrylate and 4-hydroxybutyl (meth)acrylate with at least one compound selected from the group consisting of (meth)acryloyl chloride, 2-methacryloyloxyethyl isocyanate and 2-acryloyloxyethyl isocyanate, whereby favorable reactivity with the prepolymer (A) will be obtained.

### (Unit (u3))

The liquid repellent polymer (C) may have other units (u3) other than the units (u1) and the units (u2), as the case requires, within a range not to impair the effect to improve the liquid repellency. In a case where the liquid repellent polymer (C) has the units (u4) or the units (u5), these units are regarded as the units (u3).

The units (u3) are preferably introduced to the liquid repellent polymer (C) by polymerizing a compound (m3) having a polymerizable functional group. Further, it is also preferred to introduce the units (u3) to the copolymer by reacting a compound (a3) having a functional group reactive with the reactive functional groups of the liquid repellent polymer (C).

The compound (3) may, for example, be ethyl isocyanate, propyl isocyanate, isopropyl isocyanate, butyl isocyanate, t-butyl isocyanate, hexyl isocyanate, cyclohexyl isocyanate, dodecyl isocyanate, octadecyl isocyanate, phenyl isocyanate, acetyl chloride, propionyl chloride, butyryl chloride, isobutyl chloride, pivaloyl chloride, isovaleryl chloride, valeryl chloride, 3,3-dimethylbutylryl chloride, hexanoyl chloride, heptanoyl chloride, 2-ethylhexanoyl chloride, octanoyl chloride, nonanoyl chloride, decanoyl chloride or lauroyl chloride, and is preferably ethyl isocyanate, propyl isocyanate, acetyl chloride or propionyl chloride.

The compound (m3) to give the units (u3) may, other than the compound (m4), for example, be a hydrocarbon olefin, a vinyl ether, an isopropenyl ether, an allyl ether, a vinyl ester, an allyl ester, a (meth)acrylate, a (meth)acrylamide, an aromatic vinyl compound, a chloroolefin, a conjugated diene or a fluorinated monomer other than the compound (m1). The compound (m3) may have a reactive functional group. The reactive functional group may, for example, be a hydroxy group, a carbonyl group or an alkoxy group. The compound (m3) may be used alone or in combination of two or more.

The compound (m3) may, for example, be specifically acrylic acid, methacrylic acid, methyl (meth)acrylate, ethyl (meth)acrylate, n-propyl (meth)acrylate, isopropyl (meth)acrylate, n-butyl (meth)acrylate, isobutyl (meth)acrylate, sec-butyl (meth)acrylate, tert-butyl (meth)acrylate, n-pentyl (meth)acrylate, 3-methylbutyl (meth)acrylate, n-hexyl (meth)acrylate, 2-ethyl-n-hexyl (meth)acrylate, n-octyl (meth)acrylate, cyclohexyl (meth)acrylate, isobornyl (meth)acrylate, n-decyl (meth)acrylate, (1,1-dimethyl-3-oxobutyl) (meth)acrylate, 2-acetoacetoxyethyl (meth)acrylate, 2-methoxyethyl (meth)acrylate, 2-ethoxyethyl (meth)acrylate, (meth)acrylamide, N-vinylacetamide, N-vinylformamide, N-(1,1-dimethyl-3-oxobutyl) (meth)acrylamide, N-methoxymethyl (meth)acrylamide, N,N-bis(methoxymethyl) (meth)acrylamide, styrene or RUVA-93 (manufactured by Otsuka Chemical Co., Ltd.). In view of availability, it is preferably acrylic acid, methacrylic acid, methyl (meth)acrylate, n-butyl (meth)acrylate, n-hexyl (meth)acrylate, n-octyl (meth)acrylate, cyclohexyl (meth)acrylate, isobornyl (meth)acrylate, n-decyl (meth)acrylate or RUVA-93.

The proportion of the units (u2) in the liquid repellent polymer (C) is preferably from 5 to 90 mass%, more preferably from 5 to 85 mass%, further preferably from 5 to 80 mass%, particularly preferably from 5 to 60 mass%, most preferably from 5 to 50 mass%. When the proportion of the units (u2) is at least the lower limit value of the above range, the reactivity with the prepolymer (A) or the compound (B) will be favorable. When it is at most the upper limit value of the above range, the liquid repellency of the surface of a cured film will be more favorable.

The proportion of the units (u3) in the liquid repellent polymer (C) is preferably at most 70 mass%, more preferably at most 60 mass%, particularly preferably at most 50 mass%. The lower limit value is preferably 0%. When the proportion of the units (u3) is at most the upper limit value of the above range, sufficient proportions of the units (u1) and the units (2) can be secured, and the liquid repellency of the surface of a cured film and the curing property of the curable composition will not be impaired.

In a case where the liquid repellent polymer (C) consists of the units (u1) and the units (u2), it is preferred that the content of the units (u1) is such an amount that the fluorine content in the liquid repellent polymer (C) is within the above preferred range, and the rest consists of the units (u2).

In a case where the liquid repellent polymer (C) consists of the units (u1), the units (u2) and the units (u3), it is preferred that the content of the units (u1) is such an amount that the fluorine content in the liquid repellent polymer (C) is within the above preferred range, the content of the units (u3) is within the above preferred range, and the rest consists of the units (u2).

### (Method for producing liquid repellent polymer (C))

The liquid repellent polymer (C) can be produced by polymerizing a monomer to obtain a copolymer, followed by the above modification as the case requires.

Polymerization of a monomer is preferably carried out in a solvent. Further, for polymerization of a monomer, a polymerization initiator is preferably used, and a chain transfer agent is preferably used as the case requires. When the monomer is stored, a polymerization inhibitor is preferably used as the case requires.

The solvent may, for example, be an alcohol (such as ethanol, 1-propanol, 2-propanol, 1-butanol or ethylene glycol), a ketone (such as acetone, 2-butanone, methyl isobutyl ketone or cyclohexanone), a cellosolve (such as 2-methoxyethanol, 2-ethoxyethanol or 2-butoxyethanol), a carbitol (such as 2-(2-methoxyethoxy)ethanol, 2-(2-ethoxyethoxy)ethanol, 2-(2-butoxyethoxy)ethanol), an ester (such as methyl acetate, ethyl acetate, n-butyl acetate, ethyl lactate, n-butyl lactate, ethylene glycol monomethyl ether acetate, propylene glycol monomethyl ether acetate, ethylene glycol diacetate or glycerin triacetate) or an ether (such as diethylene glycol dimethyl ether or diethylene glycol methyl ethyl ether). The solvent may be used alone or in combination of two or more.

The polymerization initiator may, for example, be a known organic peroxide, inorganic peroxide or azo compound. The organic peroxide and the inorganic peroxide may be used as a redox catalyst in combination with a reducing agent. The polymerization initiator may be used alone or in combination of two or more.

The organic peroxide may, for example, be benzoyl peroxide, lauroyl peroxide, isobutyryl peroxide, tert-butyl hydroperoxide or tert-butyl-α-cumyl peroxide.

The inorganic peroxide may, for example, be ammonium persulfate, sodium persulfate, potassium persulfate, hydrogen peroxide or a percarbonate.

The azo compound may, for example, be 2,2'-azobisisobutyronitrile, 1,1-azobis(cyclohexane-1-carbonitrile), 2,2'-azobis(2,4-dimethylvaleronitrile), 2,2'-azobis(4-methoxy-2,4-dimethylvaleronitrile), dimethyl 2,2'-azobisisobutyrate, or 2,2'-azobis(2-amidinopropane) dihydrochloride.

The chain transfer agent may, for example, be a known mercaptan or alkyl halide. The chain transfer agent may be used alone or in combination of two or more.

The mercaptan may, for example, be n-butyl mercaptan, n-dodecyl mercaptan, tert-butyl mercaptan, ethyl thioglycolate, 2-ethylhexyl thioglycolate or 2-mercaptoethanol.

The alkyl halide may, for example, be chloroform, carbon tetrachloride or carbon tetrabromide.

The polymerization inhibitor may be a known polymerization inhibitor.

The polymerization inhibitor may, for example, be specifically 2,6-di-tert-butyl-p-cresol.

In a case where the copolymer is modified also, the same solvent as above may be used. However, a solvent which may react with the compound (a2) is preferably not used. Polymerization of the monomer is carried out in a solvent, and sequentially the compound (a2) is added and reacted to obtain the liquid repellent polymer (C).

Modification of the copolymer may be carried out in the presence of a catalyst or a neutralizing agent. For example, in a case where a copolymer having hydroxy groups is reacted with a compound having an isocyanate group and a crosslinkable functional group, a tin compound or the like may be used as a catalyst.

The tin compound may, for example, be dibutyltin dilaurate, dibutyltin di(maleic acid monoester), dioctyltin dilaurate, dioctyltin di(maleic acid monoester) or dibutyltin diacetate. The tin compound may be used alone or in combination of two or more.

In a case where a copolymer having hydroxy groups is reacted with a compound having an acyl chloride group and a crosslinkable functional group, a basic catalyst may be used.

The basic catalyst may, for example, be triethylamine, pyridine, dimethylaniline or tetramethylurea. The basic catalyst may be used alone or in combination of two or more.

The liquid repellent polymer (C) of the present invention contains units (u1) based on the liquid repellent compound. Since the liquid repellent compound has a Cf group, the liquid repellent compound and the liquid repellent polymer (C) have favorable liquid repellency. Accordingly, the surface of a cured film obtained by curing a curable composition containing the liquid repellent polymer (C) repels water or oil, and even if water, oil or the like is attached thereto, it can easily be removed from the surface. The attached substance is not limited to a liquid, and may be a solid having an adherent surface.

The liquid repellent polymer (C) imparts to the surface of a cured film obtained by curing a curable composition, properties to decrease the adhesion property and properties to make removal of the attached substance easy. The liquid repellent polymer (C) is useful as a non-adhesion imparting agent to be blended in a curable composition. For example, if an oily substance (particularly e.g. finger prints) such as sebum is attached to the surface of the cured film, the attached substance can easily be removed. Even in a case where such properties are utilized, as described above, a surface on which the properties are partially decreased can be formed.

Further, the liquid repellent compound is a compound (m1) in which R³ in "a copolymerizable photoinitiator" disclosed in JP-A-62-81345 replaced with a Cf group, and accordingly like "the copolymerizable photoinitiator" disclosed in the document, decomposition occurs in its molecule by irradiation with ultraviolet light having a wavelength of from 350 to 370 nm even if no photocatalyst or the like is present, and a decomposition residue containing the Cf group can leave. That is, from the liquid repellent polymer (C), the Cf group present in the side chain is likely to leave by irradiation with ultraviolet light. Therefore, by partially irradiating the surface of a cured film obtained by curing a curable composition containing the liquid repellent polymer (C) with ultraviolet light, the liquid repellency of the portion irradiated with ultraviolet light of the surface of the cured film is decreased, and the portion may be converted to be relatively liquid-philic to the portion not irradiated with ultraviolet light.

As described hereinafter, by irradiating the surface of a cured film obtained by curing a curable composition containing the liquid repellent polymer (C) with ultraviolet light via a photomask having a pattern, the portion irradiated with ultraviolet light can be converted to be liquid-philic, and a surface having a pattern of a liquid repellent region not irradiated with ultraviolet light and a liquid-philic region can be obtained.

### [Curable composition]

The curable composition of the present invention is a composition containing the liquid repellent polymer (C).

As described hereinafter, it is a film made of a cured product obtained by heat-curing or photocuring the curable composition of the present invention. Accordingly, the curable composition of the present invention is a heat-curable composition or a photocurable composition.

The curable composition of the present invention preferably further contains a radical polymerization initiator (D) in view of the curing property. In view of the solvent resistance, the dielectric constant, etc. of an obtainable cured film, it is preferably a curable composition which further contains the prepolymer (A). In view of the hardness of an obtainable cured film, it preferably further contains the compound (B).

### (Fluorinated polyarylene prepolymer (A))

The prepolymer (A) has a polyarylene structure having a plurality of aromatic rings bonded via a single bond or a linking group, and has fluorine atoms and a crosslinkable functional group. By the curable composition containing the prepolymer (A), the dielectric constant of an obtainable cured film can be made low.

The crosslinkable functional group of the prepolymer (A) undergoes substantially no reaction at the time of producing the prepolymer (A), and undergoes radical polymerization reaction to cause crosslinking or chain extension between molecules of the prepolymer (A), by addition of an external energy.

Further, it is also reacted with the crosslinkable functional group of the compound (B) or the liquid repellent polymer (C) and integrated with it to produce a cured film. The crosslinkable functional group of the prepolymer (A) is preferably a vinyl group or an ethynyl group from the viewpoint that the reactivity at the time of producing the prepolymer (A) is low and that the reactivity in the presence of the radical polymerization initiator (D) is good.

The linking group in the polyarylene structure may, for example, be an ether bond (-O-), a sulfide bond (-S-), a carbonyl group (-CO-) or a sulfonyl group (-SO₂-).

In the prepolymer (A), a polymer having a structure in which aromatic rings are bonded by a liking group containing an ether bond (-O-) is referred to as "a fluorinated polyarylene ether prepolymer". The fluorinated polyarylene ether prepolymer is preferred in that it has an etheric oxygen atom, whereby the molecular structure has flexibility, and the flexibility of a cured film is good.

The prepolymer (A) preferably comprises a fluorinated polyarylene ether prepolymer, particularly preferably consists solely of the fluorinated polyarylene ether prepolymer.

As a specific example of the linking group containing the ether bond, an ether bond (-O-) made solely of an etheric oxygen atom or an alkylene group containing an etheric oxygen atom in a carbon chain may, for example, be mentioned.

The prepolymer (A) has fluorine atoms. As it has fluorine atoms, the dielectric constant and the dielectric loss of a cured film tend to be low, such being desirable as a material to form an insulation film. When the dielectric constant and dielectric loss of an insulation film are low, it is possible to prevent delay of a signal propagation velocity and to obtain a device excellent in electrical properties.

Further, as it has fluorine atoms, the water-absorptivity of the cured film becomes low, whereby it is possible to prevent a change in the bonded state at the bonded electrodes and wiring portions therearound, or it is possible to prevent deterioration (such as rusting) of metals, and it presents a substantial effect to improve the reliability of a device.

As a specific example of the prepolymer (A) in the present invention, a prepolymer (hereinafter, also referred to as "prepolymer (A1)") having a crosslinkable functional group and an ether bond, obtained by subjecting either one or both of a compound (x1) having a crosslinkable functional group and a phenolic hydroxy group and a compound (x2) having a crosslinkable functional group and a fluorinated aromatic ring, a compound (y) represented by the following formula (y), and a compound (z) having at least three phenolic hydroxy groups, to a condensation reaction in the presence of a hydrogen halide-removing agent, may be mentioned. 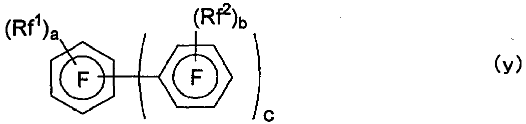 wherein a is an integer of from 0 to 3, b is an integer of from 0 to 3, and c is an integer of from 0 to 3.

Each of Rf¹ and Rf² which are independent of each other, is a fluoroalkyl group having at most 8 carbon atoms, and F in the aromatic ring represents that hydrogen atoms of the aromatic ring are all substituted by fluorine atoms.

As a preferred example of the compound (y), a compound wherein a is 0 or 1, b is 0 or 1, c is 0 or 1, Rf¹ is CF₃, and Rf² is CF₃, may be mentioned, and among them, perfluorobenzene, perfluorotoluene or perfluorobiphenyl is preferred.

The compound (z) may, for example, be 1,3,5-trihydroxybenzene, tris(4-hydroxyphenyl)methane, tris(4-hydroxyphenyl)ethane or 4-[4-[1,1-bis(4-hydroxyphenyl)ethyl]]-α,α-dimethylbenzylphenol, and is preferably 1,3,5-trihydroxybenzene or tris(4-hydroxyphenyl)ethane.

In the prepolymer (A1), by using the compound (z) having at least three phenolic hydroxy groups, it is possible to introduce branched structures to the polymer chain to make the molecular structure three dimensional thereby to increase the free volume of the polymer, whereby low densification i.e. a low dielectric constant can be accomplished.

Further, usually, a linear chain polymer having aromatic rings is likely to undergo orientation of molecules due to stacking of the aromatic rings, but with the cured product of the present invention, orientation of molecules is suppressed by the introduction of branched structures, and consequently, the birefringence will be small.

The prepolymer (A1) may be produced by either or both of the following methods (i) and (ii).
(i) A method of subjecting the compound (y), the compound (z) and the compound (x1) to a condensation reaction in the presence of a hydrogen halide-removing agent.
(ii) A method of subjecting the compound (y), the compound (z) and the compound (x2) to a condensation reaction in the presence of a hydrogen halide-removing agent.

Further, in a case where the prepolymer (A1) is produced by both of the above (i) and (ii), the compound (y), the compound (z), the compound (x1) and the compound (x2) are subjected to a condensation reaction in the presence of a hydrogen halide-removing agent.

Moreover, in each of the above methods (i) and (ii), the condensation reaction may be a single stage reaction or a multistage reaction. Further, among the reaction raw materials, a specific compound may be reacted preferentially in advance, and subsequently the other compounds may be reacted. In the case of the multistage condensation reaction, an intermediate product obtained in the middle of the reaction may be separated from the reaction system, purified, and then used for a subsequent reaction (condensation reaction). In the reaction site, the raw material compounds may be charged all together, continuously or intermittently.

In the above process for producing the prepolymer (A1), the condensation reaction proceeds as represented by the following formula (1) in which an ether bond is formed by e.g. a reaction mechanism wherein a phenoxy group derived from a phenolic hydroxy group attacks the carbon atom to which a fluorine atom is bonded of the compound (y), and then the fluorine atom is detached.

Further, in a case where the compound (z) and/or (x1) has two phenolic hydroxy groups which are in an ortho position to each other, there is a possibility that a dioxine skeleton is formed by the reaction represented by the following formula (2) by e.g. a similar reaction mechanism. From the viewpoint that the molecular structure has flexibility and the cured film has favorable flexibility, preferred is a prepolymer having no dioxine structure. That is, it is preferred that the compound (z) and/or the (x1) does not have two phenolic hydroxy groups which are in an ortho position to each other.

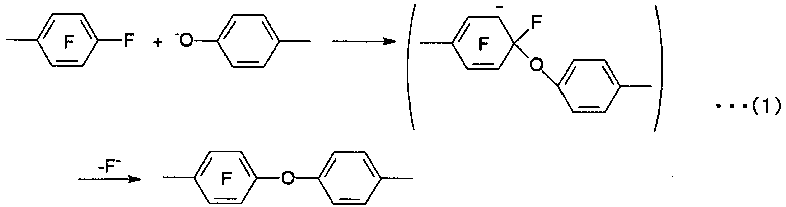

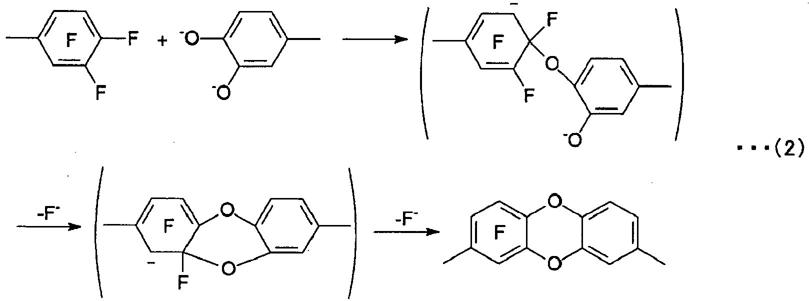

As the compound (x1) to be used in the production process (i), a compound (x11) having one phenolic hydroxy group and a compound (X12) having two phenolic hydroxy groups, are preferred.

Specific examples of the compound (x11) include a phenol having a reactive double bond such as 4-hydroxystyrene; and an ethynylphenol such as 3-ethynylphenol, 4-phenylethynyl phenol and 4-(4-fluorophenyl)ethynylphenol. They may be used alone or in combination as a mixture of two or more of them.

Specific examples of the compound (X12) include a bis(phenylethynyl)dihydroxybiphenyl such as 2,2'-bis(phenylethynyl)-5,5'-dihydroxybiphenyl and 2,2'-bis(phenylethynyl)-4,4'-dihydroxybiphenyl; and a dihydroxydiphenylacetylene such as 4,4'-dihydroxytolane and 3,3'-dihydroxytolane. They may be used alone or in combination as a mixture of two or more of them.

As the compound (x2) to be used in the production process (ii), a compound having a crosslinkable functional group and a perfluoroaromatic ring such as perfluorophenyl or perfluorobiphenyl, is preferred. Its specific examples include a fluorinated aryl having a reactive double bond, such as pentafluorostyrene, pentafluorobenzyl acrylate, pentafluorobenzyl methacrylate, pentafluorophenyl acrylate, pentafluorophenyl methacrylate, perfluorostyrene, pentafluorophenyl trifluorovinyl ether and 3-(pentafluorophenyl)pentafluoropropene-1; a fluorinated aryl having a cyano group such as pentafluorobenzonitrile; a fluorinated arylacetylene such as pentafluorophenylacetylene and nonafluorobiphenylacetylene; and a fluorinated diarylacetylene such as phenylethynylpentafluorobenzene, phenylethynylnonafluorobiphenyl and decafluorotolane. They may be used alone or in combination as a mixture of two or more of them.

As the compound (x2), a fluorinated aryl having a double bond or a fluorinated arylacetylene having a triple bond is preferred since the crosslinking reaction thereby proceeds at a relatively low temperature, and the heat resistance of a prepolymer cured product thereby obtained becomes high.

As the hydrogen halide-removing agent to be used for the production of the prepolymer (A1), a basic compound is preferred, and an alkali metal carbonate, hydrogen carbonate or hydroxide is particularly preferred. Specific examples include sodium carbonate, potassium carbonate, sodium hydrogencarbonate, potassium hydrogencarbonate, sodium hydroxide and potassium hydroxide.

With respect to the amount of the hydrogen halide-removing agent to be used, in the production process (i), it is required to be an amount of at least equimolar, preferably from 1.1 to 3 times by the molar ratio to the total number of moles of phenolic hydroxy groups in the compound (z) and the compound (x1). In the production process (ii), it is required to be an amount of at least equimolar, preferably from 1.1 to 3 times by the molar ratio to the number of moles of phenolic hydroxy groups in the compound (z).

In the production processes (i) and (ii), the condensation reaction is preferably carried out in a polar solvent. The polar solvent is preferably an aprotic polar solvent such as an amide such as N,N-dimethylacetamide, N,N-dimethylformamide or N-methylpyrrolidone; a sulfoxide such as dimethylsulfoxide; a sulfone such as sulfolane; or an ether such as diethyl ether, tetrahydrofuran, dioxane, diethylene glycol dimethyl ether, diethylene glycol diethyl ether or triethylene glycol dimethyl ether.

To the polar solvent, toluene, xylene, benzene, tetrahydrofuran, benzotrifluoride, xylenehexafluoride or the like may be incorporated within a range not to deteriorate the solubility of the prepolymer (A1) to be formed and not to adversely affect the condensation reaction. By such incorporation, the polarity (the dielectric constant) of the solvent may be changed to control the reaction rate.

The condensation reaction conditions are preferably from 10 to 200°C for from 1 to 80 hours, more preferably from 20 to 180°C for from 2 to 60 hours, particularly preferably from 50 to 160°C for from 3 to 24 hours.

In the production process (i), the amount of the compound (z) to be used is preferably from 0.1 to 1 time, particularly preferably from 0.3 to 0.6 time in a molar ratio to the compound (y). The amount of the compound (x1) to be used is preferably from 0.1 to 2 times, particularly preferably from 0.2 to 1.5 times in a molar ratio to the compound (y).

In the production process (ii), the amount of the compound (z) to be used is preferably from 0.5 to 2 times, particularly preferably from 0.6 to 1.5 times in a molar ratio to the compound (y). The amount of the compound (x2) to be used is preferably from 0.1 to 2 times, particularly preferably from 0.2 to 1.5 times in a molar ratio to the compound (y).

When the respective amounts are in such ranges, the resulting prepolymer (A1) will have a low dielectric constant and high heat resistance, such being desirable.

As the production process of the prepolymer (A1), the production process (i) or (ii) may suitably be selected depending upon the physical properties such as the heat resistance, relative dielectric constant, birefringence, and flexibility, of a cured product obtainable after the curing. For example, in a case where the production process (ii) is used, the relative dielectric constant and birefringence values of a cured product obtainable by curing the prepolymer (A1) thus produced usually tend to be low. That is, to obtain a cured product having low relative dielectric constant and birefringence values, it is preferred to produce the prepolymer (A1) by the production process (ii).

After the condensation reaction or after formed into a solution, the prepolymer (A1) is purified by a method such as neutralization, reprecipitation, extraction or filtration. The purification is preferably carried out in a state where the polar solvent preferably used during the production, is present, or in a state as dissolved or dispersed in the after-mentioned solvent (E), since the efficiency is thereby good.

In an application as an insulation film in electronic devices or an insulation film in multilayer wiring boards, a metal such as potassium or sodium derived from the hydrogen halide-removing agent and free halogen atoms are likely to cause operation failure of a transistor or corrosion of wiring, and accordingly, it is preferred to sufficiently carry out the purification.

Suitable examples of the prepolymer (A1) include a polymer obtainable by reacting a fluorinated aromatic compound (such as perfluoro(1,3,5-triphenylbenzene) or perfluorobiphenyl), a phenol compound (such as 1,3,5-trihydroxybenzene or 1,1,1-tris(4-hydroxyphenyl)ethane), and a crosslinkable functional group-containing aromatic compound (such as pentafluorostyrene, acetoxystyrene, chloromethylstyrene or pentafluorophenylacetylene), in the presence of a hydrogen halide-removing agent (such as potassium carbonate).

The number average molecular weight (Mn) of the prepolymer (A) is preferably from 1,000 to 100,000, particularly preferably from 5,000 to 50,000. When the number average molecular weight (Mn) is at least the lower limit value of the above range, the flexibility of the cured film does not tend to decrease. When it is at most the upper limit value of the above range, the curable composition can readily be purified.

The content of the prepolymer (A) in the curable composition of the present invention is preferably from 10 to 99.99 mass%, more preferably from 20 to 99,95 mass%, further preferably from 30 to 70 mass%, particularly preferably from 50 to 70 mass%. When it is at least the lower limit value of the above range, the dielectric constant of the cured film will be sufficiently low. Further, when it is at most the above upper limit value, the curable composition will easily be cured at low temperature, whereby the solvent resistance of the cured film will sufficiently be low.

In a case where the curable composition contains the compound (B), the content of the prepolymer (A) is preferably from 20 to 90 parts by mass, more preferably from 30 to 85 parts by mass, particularly preferably from 40 to 80 parts by mass based on the total amount (100 parts by mass) of the prepolymer (A) and the compound (B).

### (Compound (B))

The compound (B) is a compound having a number average molecular weight (Mn) of from 140 to 5,000, having a crosslinkable functional group and having no fluorine atoms. By the curable composition containing the compound (B), a cured film having high hardness can be formed.

The number average molecular weight (Mn) of the compound (B) is preferably from 200 to 3,000, particularly preferably from 250 to 2,500. When the number average molecular weight (Mn) is at least the lower limit value of the above range, the compound (B) is less likely to be volatilized by heating. When it is at most the upper limit value of the above range, the viscosity of the compound (B) can be suppressed to be low, and therefore a uniform curable composition can readily be obtained when the compound (B) is mixed with the prepolymer (A).

The compound (B) has preferably at least 2 crosslinkable functional groups, whereby it is capable of intermolecular crosslinking, and more preferably has from 2 to 20, particularly preferably from 2 to 8 crosslinkable functional groups.

The crosslinkable functional groups of the compound (B) are preferably groups having no fluorine atoms and being reactive in the same step as in the step in which the crosslinkable functional group of the prepolymer (A) undergoes radical polymerization reaction.

The crosslinkable functional group of the compound (B) induces crosslinking or chain extension by the reaction of at least the crosslinkable functional groups with each other. Further, it is reacted with the crosslinkable functional group of the prepolymer (A) or the liquid repellent polymer (C), and they are integrated to form a cured film.

The crosslinkable functional group of the compound (B) is preferably a (meth)acryloyl(oxy) group from the viewpoint of high reactivity and availability, particularly preferably an acryloyl(oxy) group from the viewpoint of higher reactivity. Further, two or more different crosslinkable functional groups may be present in one molecule.

Further, each of the prepolymer (A), the compound (B) and the liquid repellent polymer (C) may have two or more different crosslinkable functional groups in one molecule. Further, the crosslinkable functional groups in the prepolymer (A), the compound (B) and the liquid repellent polymer (C) coexisting in the curable composition may be the same or different.

Specific examples of the compound (B) include dipentaerythritol triacrylate triundecylate, dipentaerythritol pentaacrylate monoundecylate, ethoxylated isocyanuric acid triacrylate, ε-caprolactone-modified tris-(2-acryloxyethyl) isocyanurate, 9,9-bis[4-(2-acryloyloxyethoxy)phenyl]fluorene, polyethylene glycol diacrylate, polyethylene glycol dimethacrylate, polypropylene glycol diacrylate, polypropylene glycol dimethacrylate, ethoxylated bisphenol A diacrylate, ethoxylated bisphenol A dimethacrylate, propoxylated bisphenol A diacrylate, propoxylated bisphenol A dimethacrylate, 1,10-decanediol diacrylate, 1,6-hexanediol diacrylate, 1,6-hexanediol dimethacrylate, 1,4-butanediol dimethacrylate, 1,3-butanediol dimethacrylate, hydroxypivalic acid neopentyl glycol diacrylate, 1,9-nonanediol diacrylate, 1,9-nonanediol dimethacrylate, neopentyl glycol diacrylate, neopentyl glycol dimethacrylate, pentaerythritol triacrylate, trimethylolpropane triacrylate, ethoxylated trimethylolpropane triacrylate, propoxylated trimethylolpropane triacrylate, triallyl cyanurate, triallyl isocyanurate, trimethallyl isocyanurate, 1,4-butanediol divinyl ether, 1,9-nonanediol divinyl ether, cyclohexane dimethanol divinyl ether, triethylene glycol divinyl ether, trimethylolpropane trivinyl ether, pentaerythritol tetravinyl ether, 2-(2-vinyloxyethoxy)ethyl acrylate, 2-(2-vinyloxyethoxy)ethyl methacrylate, trimethylolpropane diallyl ether, pentaerythritol triallyl ether, dipentaerythritol hexaacrylate, pentaerythritol tetraacrylate, an ethoxylated pentaerythritol tetraacrylate represented by the following formula (B-1), a propoxylated pentaerythritol tetraacrylate represented by the following formula (B-2), ditrimethylolpropane tetraacrylate, tricyclodecane dimethanol diacrylate, tricyclodecane dimethanol methacrylate, and a compound represented by the following formula (B-3).

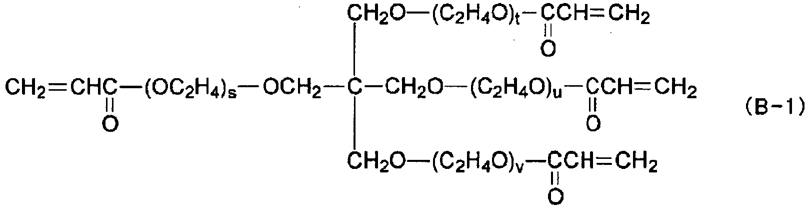

(In the formula, s+t+u+v is from 4 to 35)

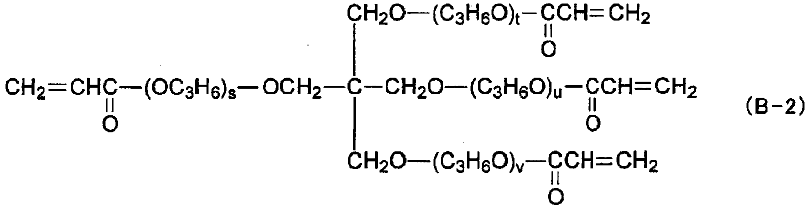

(In the formula, s+t+u+v is about 4)

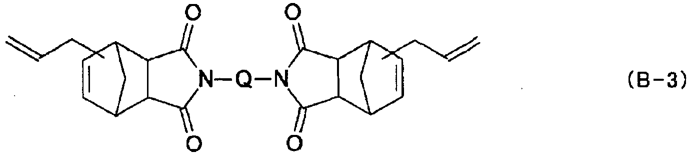

(In the formula, Q is

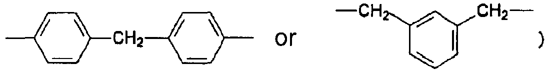

As the compound (B), polyester acrylates (a compound obtained by modifying both terminals of a condensate of a dihydric alcohol and a dibasic acid with acrylic acid, tradename: Aronix (M-6100, M-6200, M-6250 or M-6500), manufactured by TOAGOSEI CO., LTD.; and a compound obtained by modifying terminal hydroxy groups of a condensate of a polyhydric alcohol and a polybasic acid, with acrylic acid, tradename: Aronix (M-7100, M-7300K, M-8030, M-8060, M-8100, M-8530, M-8560 or M-9050) manufactured by TOAGOSEI CO., LTD.) may also be used. These products are commercially available.

As the compound (B) to be used in the present invention, ethoxylated isocyanuric acid triacrylate, 1,10-decanediol diacrylate, 1,9-nonanediol diacrylate, 1,9-nonanediol dimethacrylate, trimethylolpropane triacrylate, dipentaerythritol hexaacrylate, pentaerythritol tetraacrylate, ditrimethylolpropane tetraacrylate, tricyclodecane dimethanol diacrylate or ε-caprolactone modified tris-(2-acryloxyethyl)isocyanurate is preferred from the viewpoint of availability and reactivity.

In a case where the compound (B) is incorporated into the curable composition of the present invention, its content is preferably from 10 to 80 parts by mass, more preferably from 15 to 70 parts by mass, particularly preferably from 20 to 60 parts by mass based on the total amount (100 parts by mass) of the prepolymer (A) and the compound (B). When the content is at least the lower limit value of the above range, the curable composition is likely to be cured at low temperature, whereby the solvent resistance of the obtainable cured film will sufficiently be improved. Such a composition can be applied to low temperature process using a substrate having low heat resistance. Further, in a case where the substrate has a large area, warpage of the substrate can be prevented. When it is at most the upper limit value of the above range, the dielectric constant of an obtainable cured film will sufficiently be low.

### (Liquid repellent polymer (C))

The liquid repellent polymer (C) is the above-described liquid repellent polymer of the present invention. By incorporating the liquid repellent polymer (C) to the curable composition, the liquid repellency of the surface of an obtainable cured film will be favorable.

The content of the liquid repellent polymer (C) in the curable composition of the present invention is preferably from 0.01 to 20 mass%, more preferably from 0.05 to 10 mass%, particularly preferably from 0.1 to 5 mass%. When the content is at least the lower limit value of the above range, the liquid repellency of the surface of the cured film will be favorable. When it is at most the upper limit value of the above range, the film physical properties of the cured film will be favorable.

In a case where the curable composition of the present invention contains the prepolymer (A) and the compound (B), the content of the liquid repellent polymer (C) is preferably from 0.1 to 20 parts by mass, particularly preferably from 0.2 to 15 parts by mass based on the total amount (100 parts by mass) of the prepolymer (A) and the compound (B).

### (Radical polymerization initiator (D))

The curable composition of the present invention may be heat-curable or photocurable. In a case where it is heat-curable, the curable composition preferably contains a thermal polymerization initiator (D1) as the radical polymerization initiator (D), and in a case where it is photocurable, it preferably contains a photopolymerization initiator (D2).

The curable composition which is photocurable may be used as a negative photosensitive material.

As the thermal polymerization initiator (D1), a known initiator may be used. Specifically, it may, for example, be 2,2'-azobisisobutyronitrile, benzoyl peroxide, tert-butyl hydroperoxide, cumene hydroperoxide, di-tert-butyl peroxide or dicumyl peroxide. In view of the decomposition temperature, preferred is 2,2'-azobisisobutyronitrile or benzoyl peroxide. The thermal polymerization initiator (D1) may be used alone or in combination of two or more.

In a case where photocurable composition of the present invention contains the thermal polymerization initiator (D1), its content is preferably from 0.1 to 20 mass%, particularly preferably from 1 to 15 mass%. When the content is at least the lower limit value of the above range, such a curable composition is likely to be cured at low temperature, whereby the solvent resistance of an obtainable cured film will sufficiently be improved. When it is at most the upper limit value of the above range, the storage stability of the curable composition will be favorable.

As the photopolymerization initiator (D2), a known initiator may be used. Specifically, it may, for example, be an oxime ester derivative (such as 1,2-octanedion, 1-[4-(phenylthio)-, 2-(o-benzoyloxime)] (for example, tradename: IRGACURE OXE01 manufactured by Ciba Specialty Chemicals Corporation), ethanone, 1-[9-ethyl-6-(2-methylbenzoyl)-9H-carbazol-3-yl]-, 1-(o-acetyloxime) (such as tradename: IRGACURE OXE02, manufactured by Ciba Specialty Chemicals Corporation), an α-aminoalkylphenone type compound (such as tradename: IRGACURE 369 or IRGACURE 907, manufactured by Ciba Specialty Chemicals Corporation), an acylphosphineoxide type compound (such as tradename: DAROCUR TPO, manufactured by Ciba Specialty Chemicals Corporation). From the viewpoint of reactivity of radicals to be generated, preferred is IRGACURE OXE01 or IRGACURE OXE02.

In a case where the curable composition of the present invention contains the photopolymerization initiator (D2), its content is preferably from 0.1 to 20 mass%, particularly preferably from 1 to 15 mass%. When the content is at least the lower limit value of the above range, such a composition is likely to be cured at low temperature, whereby the solvent resistance of an obtainable cured film will sufficiently be improved. When it is at most the upper limit value of the above range, the storage stability of the curable composition will be favorable.

### (Additives)

To the curable composition, an additive selected from various additives well known in the field of coating, for example, stabilizers (such as an ultraviolet absorber, an antioxidant, a thermal polymerization preventing agent, etc.), surfactants (such as a leveling agent, a defoaming agent, a precipitation-preventing agent, a dispersant, etc.), plasticizers and thickeners, may be incorporated, as the case requires, so as not to impair the effects of the present invention.

In a case where the cured film is a material (for example, an interlayer insulation film) which remains as a member which functions in a final product without being removed during the production process, an adhesion-improving agent (such as a silane coupling agent) may be added to the curable composition. It is preferred to incorporate an adhesion-improving agent to the curable composition, since the adhesion between the cured film of the curable composition and a layer adjacent thereto will be improved. It is possible to improve adhesion also by a method of preliminarily applying an adhesion-improving agent to the layer adjacent thereto.

In a case where an additive is contained in the curable composition of the present invention, its content is preferably from 0.0001 to 30 mass%, particularly preferably from 0.0001 to 20 mass%.

### (Preferred combination of curable composition)

Composition 1: A curable composition comprising the following prepolymer (A), compound (B), liquid repellent polymer (C) and thermal polymerization initiator (D1).

Prepolymer (A): A prepolymer made of perfluorobiphenyl, 1,3,5-trihydroxybenzene and acetoxystyrene. The content of the prepolymer (A) in the curable composition is from 30 to 70 parts by mass.

Compound (B): At least one member selected from the group consisting of ethoxylated isocyanuric acid triacrylate, ε-caprolactone-modified tris-(2-acryloxyethyl) isocyanurate, 1,10-decanediol diacrylate, 1,9-nonanediol diacrylate, 1,9-nonanediol dimethacrylate, trimethylolpropane triacrylate, dipentaerythritol hexaacrylate, pentaerythritol tetraacrylate, ditrimethylolpropane tetraacrylate and tricyclodecanedimethanol diacrylate. The amount of the compound (B) is from 20 to 60 parts by mass based on the total amount (100 parts by mass) of the prepolymer (A) and the compound (B).

Liquid repellent polymer (C): A copolymer having units (u1) and units (u2) or a copolymer having units (u1), units (u2) and units (u3), wherein the units (u1) are the after-mentioned (u1-1) to (u1-3), the units (u2) are the after-mentioned (u2-1), and the units (u3) are the after-mentioned (u3-1) to (u3-7). The content of the liquid repellent polymer (C) in the curable composition is from 0.1 to 5 parts by mass.

Thermal polymerization initiator (D1): At least one member selected from the group consisting of benzoyl peroxide and 2,2'-azobisisobutyronitrile. The content of the thermal polymerization initiator (D1) in the curable composition is from 1 to 15 parts by mass.

Combination 2: A curable composition comprising the following prepolymer (A), compound (B), liquid repellent polymer (C) and photopolymerization initiator (D2).

Prepolymer (A): A prepolymer made of perfluorobiphenyl, 1,3,5-trihydroxybenzene and acetoxystyrene. The content of the prepolymer (A) in the curable composition is from 30 to 70 parts by mass.

Compound (B): At least one member selected from the group consisting of ethoxylated isocyanuric acid triacrylate, ε-caprolactone-modified tris-(2-acryloxyethyl) isocyanurate, 1,10-decanediol diacrylate, 1,9-nonanediol diacrylate, 1,9-nonanediol dimethacrylate, trimethylolpropane triacrylate, dipentaerythritol hexaacrylate, pentaerythritol tetraacrylate, ditrimethylolpropane tetraacrylate and tricyclodecanedimethanol diacrylate. The amount of the compound (B) is from 20 to 60 parts by mass based on the total amount (100 parts by mass) of the prepolymer (A) and the compound (B).

Liquid repellent polymer (C): A copolymer having units (u1) and units (u2) or a copolymer having units (u1), units (u2) and units (u3), wherein the units (u1) are the after-mentioned (u1-1) to (u1-3), the units (u2) are the after-mentioned (u2-1), and the units (u3) are the after-mentioned (u3-1) to (u3-7). The content of the liquid repellent polymer (C) in the curable composition is from 0.1 to 5 parts by mass.

Photo polymerization initiator (D2): At least one member selected from the group consisting of IRGACURE OXE01 (manufactured by Ciba Specialty Chemicals Corporation), IRGACURE OXE02 (manufactured by Ciba Specialty Chemicals Corporation), IRGACURE 369 (manufactured by Ciba Specialty Chemicals Corporation), IRGACURE 907 (manufactured by Ciba Specialty Chemicals Corporation) and DAROCUR TPO (manufactured by Ciba Specialty Chemicals Corporation). The content of the photopolymerization initiator (D2) in the curable composition is from 1 to 15 parts by mass.

### [Coating composition]

The coating composition of the present invention contains the above curable composition and a solvent (E). The coating composition is applied on the surface of a substrate to form a coating film, and the solvent (E) is removed from the coating film. Removal of the solvent (E) is usually carried out by evaporating the solvent (E). Accordingly, it is necessary that the solvent (E) has a boiling point lower than those of components other than the solvent (E) in the curable composition. Among the above components (A) to (D), a compound having the lowest boiling point is usually the compound (B), and therefore in a case where the curable composition contains the compound (B), a solvent (E) having a boiling point lower than that of the compound (B) is used. In other words, as the compound (B), it is preferred to use a compound having a boiling point sufficiently higher than that of the solvent (E) to be used.

As the solvent (E), a known solvent may be used. A ketone type solvent, an ester type solvent, an ether type solvent, an amide type solvent or an aromatic type solvent may, for example, be mentioned, and specific examples include polypropylene glycol monomethyl ether acetate (hereinafter also referred to as "PGMEA"), mesitylene, N,N-dimethylacetamide, cyclohexanone and tetrahydrofuran.

The content of the solvent (E) in the coating composition is preferably from 1 to 99.995 mass%, more preferably from 30 to 99.99 mass%, particularly preferably from 50 to 90 mass%.

### (Preferred combination of coating composition)

As the coating composition of the present invention, the following combinations are preferred.

Combination 3: A coating composition comprising the above preferred combination 1 of the curable composition and the following solvent (E).

Solvents (E): At least one member selected from the group consisting of PGMEA and cyclohexanone. The content in the coating composition is from 50 to 90 mass%.

Combination 4: A coating composition comprising the above preferred combination 2 of the curable composition and the above solvent in the preferred combination 3 of the coating composition.

Further, in a case where the curable composition of the present invention is a curable composition containing the prepolymer (A), the compound (B), the liquid repellent polymer (C) and the radical polymerization initiator (D), it can be sufficiently cured by heating to at most 250°C (preferably at most 200°C). Accordingly, such a coating composition can be applied to a process (low temperature process) in which the upper limit value of the heating temperature is 250°C. Further, from such a coating composition, a cured film having excellent solvent resistance, a low dielectric constant and favorable liquid repellency on its surface can be formed.

### [Article having cured film]

The article having a cured film of the present invention is an article comprising a substrate, and a cured film obtained by curing the curable composition of the present invention formed on the surface of the substrate.

"The cured film obtained by curing the curable composition of the present invention formed on the surface of the substrate" includes both a case where the cured film is directly formed on the surface of a substrate and a case where an optional layer is formed on the surface of a substrate and the cured film is formed on the optional layer.

### (Substrate)

A material for the substrate may, for example, be plastic, glass or silicon. It is preferred to use plastic such as polycarbonate, polyethylene terephthalate, polyethylene naphthalate, polyethersulfone or polyimide, in view of excellent mechanical flexibility.

### (Cured film)

The thickness of the cured film may properly be set depending upon the purpose of use, and is usually at a level of from 0.1 to 100 µm, preferably from 0.2 to 50 µm.

### [Process for producing article having cured film]

The process for producing an article having a cured film of the present invention is a process comprising the following step (I).
(I) A step of applying the coating composition of the present invention on the surface of a substrate, and removing the solvent (E), followed by heating or light irradiation to form a cured film.

"Applying the coating composition of the present invention on the surface of a substrate, and removing the solvent (E), followed by heating or light irradiation to form a cured film" includes both a case where the cured film is directly formed on the surface of a substrate and a case where an optional layer is formed on the surface of a substrate and the cured film is formed on the surface of the optional layer.

In a case where the curable composition is used without using the coating composition, the following step is employed.

The curable composition of the present invention is applied on the surface of a substrate to form a coating film, followed by heating or light irradiation to form a cured film.

### (Step (I))

The application method is not particularly limited so long as a uniform coating film can be formed. For example, a spin coating method, a wipe coating method, a spray coating method, a squeegee coating method, a dip coating method, a die coating method, an ink jet method, a flow coating method, a roll coating method, a casting method, a slit coating method, a screen printing method, a Langmuir-Blodgett method or a gravure coating method may be mentioned. In view of the productivity, a spin coating method, an ink jet method or a slit coating method is preferred.

As a method of removing the solvent (E) in the coating film, a known method is mentioned, and a method by heating, a method by reducing pressure or a method by heating and reducing pressure may, for example, be mentioned. From the viewpoint that a defect is less likely to occur in the coating film, a method by heating is preferred. A heating temperature is preferably from 30 to 200°C, particularly preferably from 40 to 150°C.

In a case where the curing is carried out by heating (heat-curing), the coating composition is applied on the surface of a substrate to form a coating film, a heating step (prebaking) for the purpose of removing the solvent (E) is carried out, and further a heating step (curing step) is carried out to obtain a cured film. In the case of heat-curing, the heating step for curing may also function as the heating step for removing the solvent.

A heating temperature of the heating step (prebaking) is preferably from 40 to 200°C, particularly preferably from 60 to 200°C.

A heating temperature of the heating step (curing step) is preferably from 100 to 200°C, particularly preferably from 120 to 200°C.

A heating time of the heating step (prebaking) is preferably from 1 to 10 minutes, particularly preferably from 1 to 5 minutes.

A heating time of the heating step (curing step) is preferably from 1 to 10 minutes, particularly preferably from 1 to 5 minutes.

Here, "the heating temperature is at most 200°C" means that the temperature of an article to be heated does not exceeds 200°C. Substantially, a temperature of a heating device such as a hotplate or an oven may be set to at most 200°C.

In a case where the curing is carried out by irradiation with light (photocuring), the coating composition is applied on the surface of a substrate to form a coating film, a heating step (prebaking) for the purpose of removing the solvent (E) is carried out, then the film is irradiated (exposed) with light, and as the case requires, a heating step (curing step) is carried out to obtain a cured film.

Light to be applied for curing the film is not particularly limited so long as it is light having a wavelength to which the photopolymerization initiator (D2) contained in the curable composition has a sensitivity. Usually, light to be used for curing is ultraviolet light (wavelength: 200 to 400 nm, preferably 300 to 400 nm), but is not limited thereto. Further, it is preferred not to use light having a wavelength with which decomposition occurs in the side chains of the units (u1) in the liquid repellent polymer (C), and the photopolymerization initiator (D2) having a sensitivity to light having such a wavelength.

A heating temperature of the heating step (prebaking) in the case of photocuring is preferably from 30 to 100°C, particularly preferably from 40 to 100°C.

A heating temperature of the heating step (curing step) is preferably from 60 to 200°C, particularly preferably from 100 to 200°C.

A heating time of the heating step (prebaking) is preferably from 1 to 20 minutes, particularly preferably from 1 to 10 minutes.

A heating time of the heating step (curing step) is preferably from 1 to 20 minutes, particularly preferably from 1 to 10 minutes.

In the case of carrying out micro-fabrication by photolithography, by selective irradiation (exposure) with light using a photomask or laser, the irradiated portion (exposed portion) is cured. Accordingly, after the exposure, development (a step of dissolving or dispersing the non-exposed portion in a solvent and removing it) is carried out to remove the non-exposed portion, and the remaining solvent in the cured portion is removed to obtain a micro-fabricated cured film. As the case requires, after the development, a heating step (curing step) may be carried out. In such a case, the remaining solvent can be removed in this heating step (curing step). Further, after the exposure and before the development, as the case requires, a heating step (post-exposure baking) may be carried out.

The heating temperature of the heating step (post-exposure baking) is preferably from 60 to 200°C, particularly preferably from 100 to 200°C. The heating time of the heating step (post-exposure baking) is preferably from 1 to 20 minutes, particularly preferably from 1 to 10 minutes.

The process for producing an article having a cured film of the present invention may further have the step (II) after the above step (I). By the process having the step (II), patterning of a liquid-philic region and a liquid repellent region can be carried out.
(II) A step of partially irradiating the surface of the cured film obtained in the above step (I) with ultraviolet light to obtain an article having a pattern of a liquid-philic region and a liquid repellent region on the surface of the cured film.

Hereinafter, a portion not irradiated with ultraviolet light will be referred to as a liquid repellent region, and a portion converted to be liquid-philic by irradiation with ultraviolet light will be referred to as a liquid-philic region.

To form a pattern of a liquid-philic region and a liquid repellent region, a method of irradiating the surface of the cured film with ultraviolet light via a photomask, or a method of selectively irradiating the surface of the cured film with ultraviolet light by using laser, may be mentioned.

As a light source of the ultraviolet light, a light source which can apply ultraviolet light having a wavelength of at least 300 nm such as a high-pressure mercury lamp (i-line: 365 nm) or a YAG laser (third harmonic: 355 nm) may be used. Since the surface of the cured film may be converted to be liquid-philic also by ultraviolet light having a wavelength less than 300 nm, a light source which can apply ultraviolet light having a wavelength less than 300 nm may be used.

After the step (II), a decomposition residue containing the Cf group may be removed. For example, it may be removed by heating or in vacuum.

### [Article having functional thin film]

In the article having a pattern of a liquid-philic region and a liquid repellent region of the present invention, a functional thin film may be formed on the surface of the liquid-philic region.

As an embodiment of the functional thin film, a electrode (thin-film transistor, organic EL device), a semiconductor layer (thin-film transistor, organic EL device), a conductor layer (printed circuit board, multilayer wiring, touch panel, solar cell), a transistor material or a resin layer may, for example, be mentioned. Since the cured film is insulating, preferred is a thin-film transistor in which the functional thin-film is an electrode.

As the semiconductor layer, an organic semiconductor, an oxide semiconductor or a silicon semiconductor may be mentioned.

As the resin layer, a thermosetting resin such as a phenol resin, a urea resin, a melamine resin, an acrylic resin, an epoxy resin, a polyurethane, a polyester, a silicon resin or a polyimide, a photocurable resin or the prepolymer (A) may, for example, be mentioned.

### (Organic thin-film transistor)

Now, an example of an organic thin-film transistor will be described with reference to a drawing.

However, the functional thin film of the present invention is by no means restricted to the following organic thin-film transistor.

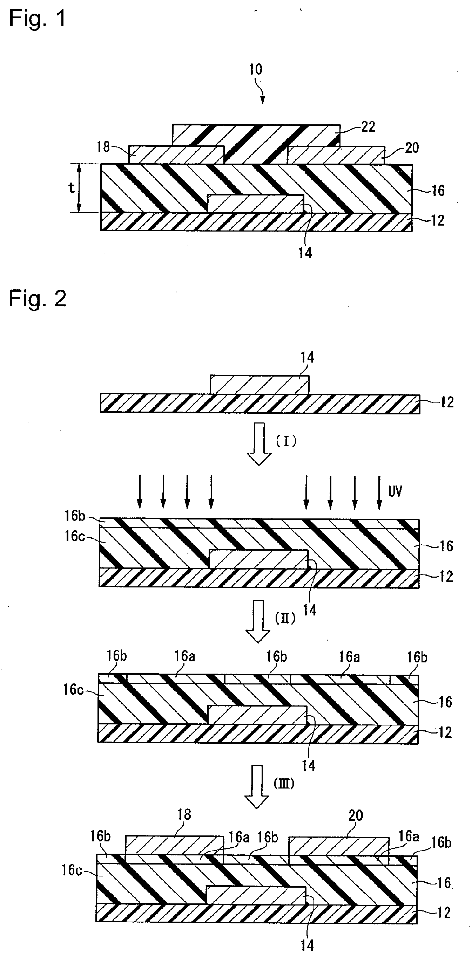

Fig. 1 is a cross-sectional view illustrating an example of an organic thin-film transistor.

An organic thin-film transistor 10 comprises a substrate 12; a gate electrode 14 formed on the surface of the substrate 12; a gate insulation film 16 (cured film) covering the surface of the gate electrode 14 and the substrate 12; a source electrode 18 and a drain electrode 20 (functional thin film) selectively formed on the surface of the gate insulation film 16; and an organic semiconductor layer 22 formed on the surface of the source electrode 18 and the drain electrode 20, and the gate insulation film 16 between the electrodes.

As a material of the substrate 12, the above-described material may be mentioned, and preferred embodiments of the substrate 12 are also the same.

The gate electrode 14, the source electrode 18 and the drain electrode 20 are formed by a conductor. The conductor may, for example, be silicon, doped silicon, platinum, gold, silver, copper, chromium, aluminum, calcium, barium, indium tin oxide, indium zinc oxide, zinc oxide, carbon black, a fullerene, carbon nanotubes, polythiophene, polyethylene dioxythiophene, a polystyrenesulfone, polyaniline, polypyrrole or polyfluorene. The conductors may be used alone or combination of two or more. The materials of the gate electrode 14, the source electrode 18 and the drain electrode 20 may be the same or different.

The gate insulation film 16 is made of a cured film obtained by curing the curable composition of the present invention on the surface of the gate electrode 14 and the substrate 12.

The thickness (the thickness t at a portion where the gate electrode 14 is not present) of the gas insulation film 16 is preferably from 1 nm to 10 µm, more preferably from 2 nm to 5 µm, particularly preferably from 5 nm to 1 µm. When the thickness of the gate insulation film 16 is at least the lower limit value of the above range, leakage current is less likely to occur between the gate electrode 14 and the source electrode 18. When it is at most the upper limit value of the above range, the driving voltage can be suppressed.

As the material of the organic semiconductor layer 22, a known low molecular weight compound, oligomer or polymer may, for example, be mentioned.

The low molecular weight compound may, for example, be pentacene, rubrene, phthalocyanine, perylene, fullerene or a derivative thereof.

The oligomer may, for example, be oligothiophene or a derivative thereof.

The polymer may, for example, be poly-p-phenylenevinylene (PPV), polyfluorene, a fluorene/benzothiadiazole copolymer, a fluorene/triphenylamine copolymer, a fluorene/dithiophene copolymer, polythiophene, polyaniline, polyacetylene, polypyrrole or derivative thereof.

The thickness of the organic semiconductor layer 22 is preferably from 5 nm to 100 µm, more preferably from 10 nm to 10 µm, particularly preferably from 10 nm to 1 µm.

In the organic thin-film transistor 10, by forming the gate insulation film 16 using the curable composition of the present invention, the leakage current is reduced. Further, since it is possible to make the gate insulation film 16 thin, downsizing of a device can be realized, and the driving voltage of the transistor can be decreased.

Further, in the organic thin-film transistor 10, since the surface of the gate insulation film 16 has favorable liquid repellency, such effects will be obtained that molecules in the organic semiconductor layer 22 provided on the gate insulation film 16 are likely to be aligned, polar groups to be top sites of a carrier are less likely to be present on the surface, and moisture and the like in the air are less likely to be adsorbed. Accordingly, the electron mobility in the organic thin-film transistor 10 will be high, and the stability and the reliability will improve.

### [Process for producing article having functional thin film]

The process for producing an article having a functional thin film of the present invention is a process comprising the above processes (I) and (II) and the following step (III).
(III) A step of selectively depositing a composition for forming a functional thin film to the surface of the above liquid-philic region (the portion of the cured film irradiated with ultraviolet light) to form a functional thin film.

The composition for forming a functional thin film is a composition for forming e.g. an electrode, a semiconductor layer, a conductor layer, a transistor material or a resin layer (hereinafter sometimes referred to as "a composition for an electrode, a composition for a semiconductor layer, a composition for a conductor layer, a composition for a transistor material or a composition for a resin layer").

The composition for forming a functional thin film may, for example, in a case where the functional film is an electrode, be a coating fluid containing the above conductor or a precursor of the conductor.

"Selectively depositing a composition for forming a functional thin film to the surface of the liquid-philic region (the portion of the cured film irradiated with ultraviolet light) to form a functional thin film" includes both a case where a functional thin film is directly formed selectively on the surface of the cured film and a case where an optional layer is formed selectively on the surface of the cured film and the cured film is formed on the surface of the optional layer.

If the composition for forming a functional thin film is applied to the surface of the cured film having a pattern of the liquid-philic region and the liquid repellent region, the composition for forming a functional thin film is selectively deposited to the liquid-philic region and will not be deposited to the liquid repellent region. Accordingly, a functional thin film (such as an electrode) having a predetermined pattern can easily be formed only on the surface of the liquid-philic region of the cured film. To form the composition for forming a functional thin film deposited to the surface of the liquid-philic region of the cured film into a functional thin film, a known method may be employed.

A functional thin film may further be formed on the surface of the liquid repellent region of the cured film of the present invention. In such a case, in order to improve the film-forming property of the functional thin film, the entire surface may be exposed without using a photomask to convert the liquid repellent region to be liquid-philic, and then the composition for forming a functional thin film to form a functional thin film may be applied to form a functional thin film.

### (Process for producing organic thin-film transistor)

Now, an example of the process for producing an organic thin-film transistor will be described with reference to the drawing.

Further, the process for producing an article having a functional thin film of the present invention is a process comprising the above steps (I) to (III) and is not limited to the following process for producing an organic thin-film transistor.

### Step (I):

As shown in Fig. 2, a substrate 12 having a gate electrode 14 formed on its surface is prepared.

As a method for forming the gate electrode 14, sputtering, vacuum deposition, spin coating, spray coating, printing or ink jet may, for example, be mentioned.

Then, the coating composition of the present invention is applied to the surface of the gate electrode 14 and the substrate 12, and the solvent (E) is removed, followed by heating or light irradiation to form a gate insulation film 16 (cured film). It is considered that in the gate insulation film 16, structures derived from the prepolymer (A) gather on the substrate 12 side, and the Cf groups gather on the side opposite from the substrate 12. That is, the surface of the gate insulation film 16 is a liquid repellent region 16b having liquid repellency. The reference symbol 16c in the drawing illustrates an internal region other than the surface of the gate insulation film 16.

### Step (II):

As shown in Fig. 2, the surface of the gate insulation film 16 is partially irradiated with ultraviolet light (UV) using a photomask (not shown) or laser (not shown) to make at least some of the Cf groups on the surface of the gate insulation film 16 leave to form a liquid-philic region 16a. In such a manner, a pattern of the liquid-philic region 16a and the liquid repellent region 16b is formed. It is considered that in the gate insulation film 16, the liquid-philic region 16a and the liquid repellent region 16b are not distinctly separated from the internal region 16c below their surfaces having surface properties, but the concentration of the Cf groups varies continuously in the thickness direction.

### Step (III):

A coating fluid containing a conductor or a precursor of the conductor is applied to the surface of the gate insulation film 16 (cured film) having a pattern of the liquid-philic region 16a and the liquid repellent region 16b, whereupon the coating fluid is selectively deposited to the liquid-philic region 16a and is not deposited to the liquid repellent region 16b. Accordingly, as shown in Fig. 2, a source electrode 18 and a drain electrode 20 having a predetermined pattern can easily be formed only on the liquid-philic region 16a of the gate insulation film 16.

As a method of applying the coating fluid, ink jet, a dispenser, printing or the like may be mentioned.

### Step (IV):

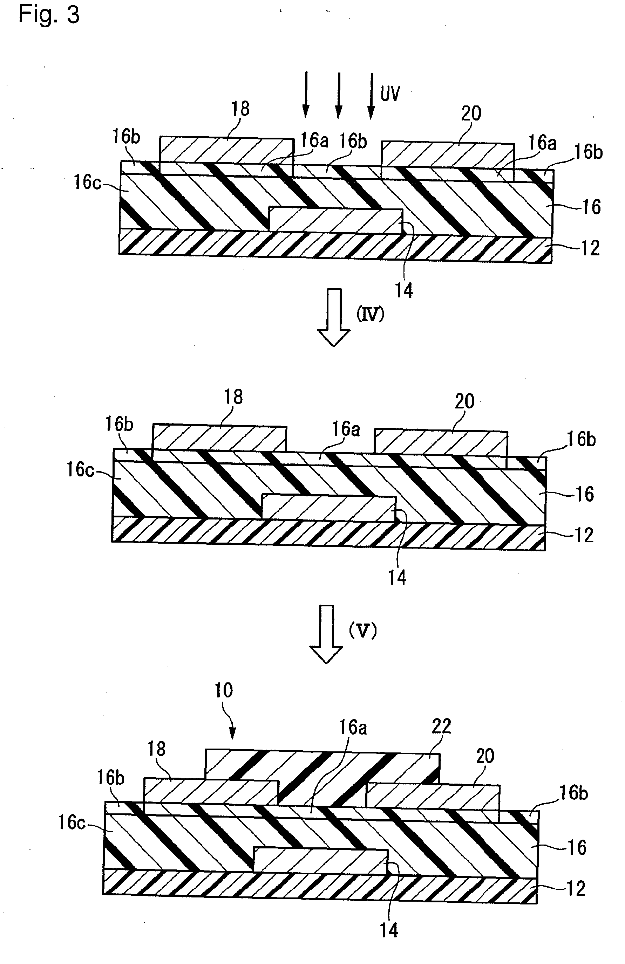

As shown in Fig. 3, at least the liquid repellent region 16b of the gate insulation film 16 between the source electrode 18 and the drain electrode 20 is irradiated with ultraviolet light (UV) to make at least some of the Cf groups on the surface of the gate insulation film 16 leave to convert the surface of the gate insulation film 16 between the electrodes to a liquid-philic region 16a. By converting the surface of the gate insulation film 16 between the electrodes to a liquid-philic region 16a, the following composition for forming an organic semiconductor layer can be applied to the surface of the gate insulation film 16 between the electrodes.

### Step (V):

As shown in Fig. 3, a composition for forming an organic semiconductor layer is applied to the surface of the source electrode 18 and the drain electrode 20, and the gate insulation film 16 between the electrodes, and an organic semiconductor layer 22 is formed by a known method such as heating. Further, a method of forming a layer made of a precursor of an organic semiconductor and then converting the precursor to the organic semiconductor by applying light or heat may also be employed. The precursor may, for example, be silylethyne-substituted pentacene or a tetrabicycloporphyrin derivative. The precursor may be converted to pentacene or a tetrabenzoporphyrin derivative by heating.

### EXAMPLES

Now, the present invention will be described in further detail with reference to Examples. However, it should be understood that the present invention is by no means restricted to such specific Examples.

Example 1 is a Preparation Example and Examples 2 to 31 are Examples of the present invention.

### (PGMEA contact angle)

The contact angle of the surface of the cured film was measured by a droplet method at 25°C using a contact angle meter CA-A, tradename, manufactured by Kyowa Interface Science Co., Ltd. About 1 µL of PGMEA was dropped on the cured film, and the contact angle was measured.

### [Example 1]

### (Preparation of prepolymer (A1-1))

Into a 10 L (liter) glass four-necked flask equipped with a Dimroth condenser, a thermocouple thermometer and a mechanical stirrer, perfluorobiphenyl (650 g), 1,3,5-trihydroxybenzene (117 g) and N,N-dimethylacetamide (hereinafter sometimes referred to as "DMAc") (6,202 g) were charged. The flask was heated on an oil bath with stirring, and at the time when the liquid temperature reached 60°C, sodium carbonate (575 g) was quickly added. While stirring was continued, the mixture was heated at 60°C for 24 hours. Then, the mixture was cooled to 0°C with stirring, 4-acetoxystyrene (200 g) and potassium hydroxide (532 g) were added, followed by stirring at 0°C for 24 hours, and about 10 L of 5N aqueous hydrochloric acid solution was gradually dropwise added for reprecipitation. The precipitate was collected by filtration and washed twice with pure water. Then, the precipitate was vacuum-dried at 60°C for 12 hours to obtain a white powdery prepotymer (A1-1) (800 g). The number average molecular weight (Mn) of the prepolymer (A-1) was 10,000.

### [Example 2]

### (Preparation of compound (m1-1))

2.5 g of compound (a1-1) (manufactured by Ciba Specialty Chemicals, tradename: IRGACURE 2959) and 2.3 g of triethylamine were dissolved in 62 mL of dichloromethane to obtain a solution. While the solution was stirred at 0°C, a solution having 1.0 g of acrylic acid chloride (compound (b1-1)) dissolved in 8 mL of dichloromethane was dropwise added to the solution. After completion of dropwise addition, the solution was stirred at 0°C for 1 hour to obtain a solution containing compound (c1-1).

While the solution containing compound (c1-1) was stirred at 0°C, 1.7 g of triethylamine was added. Then, 4.4 g of 2-(heptafluoropropoxy)-2,3,3,3-tetrafluoropropionic acid fluoride (compound d1-1) was dropwise added. After completion of dropwise addition, the solution was stirred at room temperature for 1 hour to obtain a solution containing compound (m1-1).

The solution containing compound (m1-1) was poured into ice water, followed by extraction three times with ethyl acetate. Then, the resulting organic layer was dried over magnesium sulfate to remove the solvent, and the residue was purified by silica gel column chromatography (eluent: ethyl acetate/hexane=1/2 (volume ratio)) to obtain 2.0 g of compound (m1-1).

Compound (m1-1) was identified by ¹H-NMR and ¹⁹F-NMR.
NMR spectra of compound (m1-1):
¹H-NMR(300.4MHz, solvent:CDCl₃, standard:TMS)δ(ppm):1.85(s, 6H), 4.27(t, J=4.8, 2H), 4.52(t, J=4.8, 2H), 5.87(dd, J=10.5, 10.8, 1 H), 6.16(dd, J=10.5, 17.1, 1 H), 6.45(dd, J=17.1, 17.4, 1H), 6.92(d, J=9.0, 2H), 7.90(d, J=9.0, 2H).
¹⁹F-NMR(282.7MHz, solvent:CDCl₃, standard:CFCl₃)δ(ppm):-132.0(m, 1F),-129.6(m, 2F), -85.4(m, 1 F), -81.2(m, 6F), -79.5(m, 1 F).

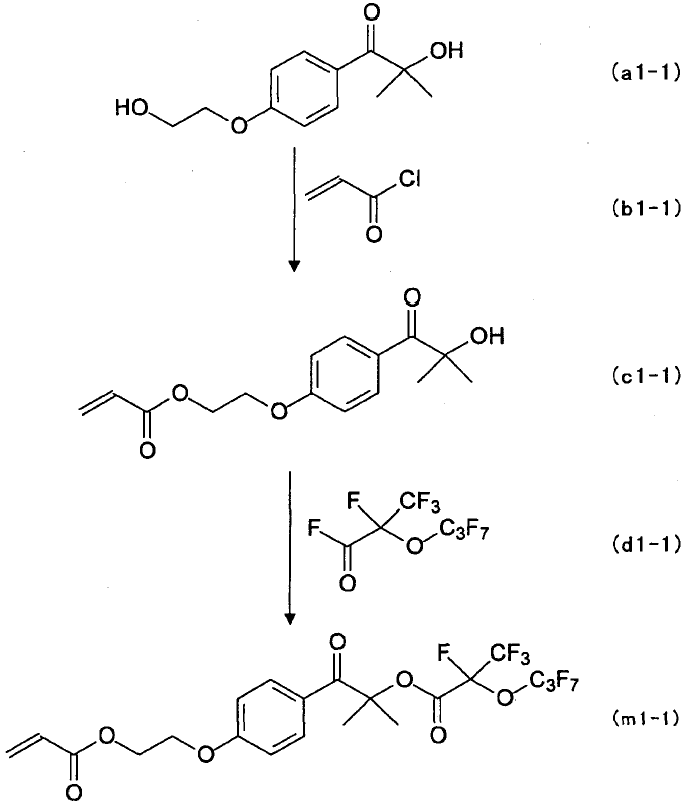

### [Example 3]

### (Preparation of liquid repellent polymer (C-1))

In 2.0 g of 2-butanone, 0.70 g of compound (m1-1) and 0.15 g of 2-hydroxyethyl methacrylate were reacted at 50°C for 24 hours in the presence of 0.034 g of n-dodecylmercaptan and 0.005 g of 2,2'-azobis(4-methoxy-2,4-dimethylvaleronitrile) (manufactured by Wako Pure Chemical Industries, Ltd., tradename: V-70). After the reaction mixture was cooled to room temperature (20 to 25°C), 0.17 g of 2-acryloyloxyethyl isocyanate, 0.0007 g of dibutyltin dilaurate and 0.0084 g of 2,6-di-tert-butyl-p-cresol were added, followed by reaction at 40°C for 24 hours to obtain a 2-butanone solution of liquid repellent polymer (C-1) having the following units (u1-1) and units (u2-1). The obtained 2-butanone solution of liquid repellent polymer (C-1) was poured into hexane to precipitate a solid, which was vacuum-dried to obtain 0.75 g of powdery liquid repellent polymer (C-1). Of liquid repellent polymer (C-1), the fluorine content was 24.2% and the number average molecular weight (Mn) was 8,000.

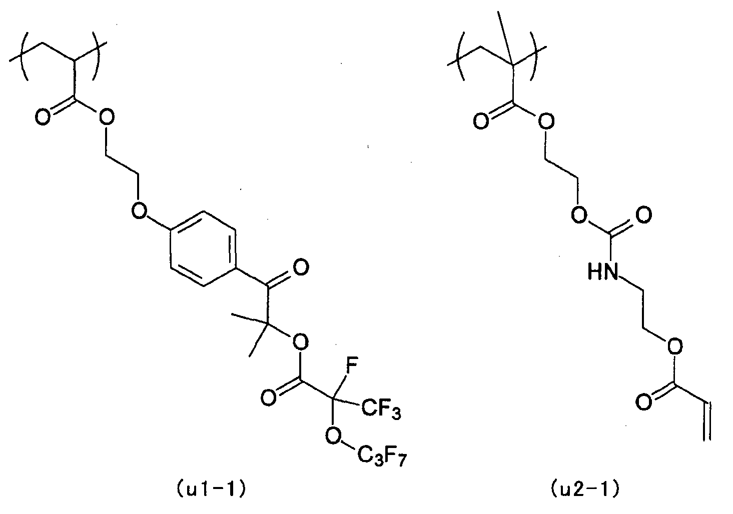

### [Example 4]

### (Preparation of coating composition)

1.2 g of prepolymer (A1-1), 0.8 g of dipentaerythritol hexaacrylate (number average molecular weight (Mn): 578) as the compound (B), 0.02 g of liquid repellent polymer (C-1) and 0.2 g of benzoyl peroxide as the thermal polymerization initiator (D1) were dissolved in 8.0 g of PGMEA to prepare a coating composition.

### [Example 5]

### (Production of article having cured film)

The coating composition was applied to a glass substrate (manufactured by Corning Incorporated, 50 mm x 50 mm x 0.725 mm in thickness) by spin coating at 1,000 revolutions per minute for 30 seconds, and heated by a hot plate at 150°C for 20 minutes to form a cured film having a thickness of 1 µm.

The surface of the cured film was partially irradiated with ultraviolet light (i-line: 365 nm) by means of a mask pattern. Irradiation with ultraviolet light was carried out using MA-8, tradename, manufactured by SUSS under irradiation conditions of 100 J/cm². By this apparatus under these conditions, ultraviolet light having a wavelength of at most 350 nm is not applied. The PGMEA contact angle of the portion irradiated with ultraviolet light was at most 10°, and the PGMEA contact angle of the portion not irradiated with ultraviolet light was 53°.

### [Example 6]

### (Preparation of compound (m1-2))

2.0 g of compound (m1-2) was obtained in the same manner as in Example 2 except that compound (b1-1) was changed to 1.2 g of methacrylic acid chloride, and compound (d1-1) was changed to 4.6 g of 2,2-difluoro-2-(1,1,2,2-tetrafluoro-2-(perfluoroethoxy)ethoxy)acetyl fluoride (compound (d1-2)).

Compound (m1-2) was identified by ¹H-NMR and ¹⁹F-NMR.
NMR spectra of compound (m1-2):
¹H-NMR(300.4MHz, solvent:CDCl₃, standard:TMS)δ(ppm):1.85(s, 6H), 1.95(s, 3H), 4.27(t, J=4.8, 2H), 4.52(t, J=4.8, 2H), 5.60(s, 1 H), 6.13(s, 1 H), 6.92(d, J=8.7, 2H), 7.92(d, J=8.7, 2H).
¹⁹F-NMR(282.7MHz, solvent:CDCl₃, standard:CFCl₃)δ(ppm):-88.9(m, 4F), -88.7(m, 2F), -87.1(m, 3F), -78.5(m, 2F).

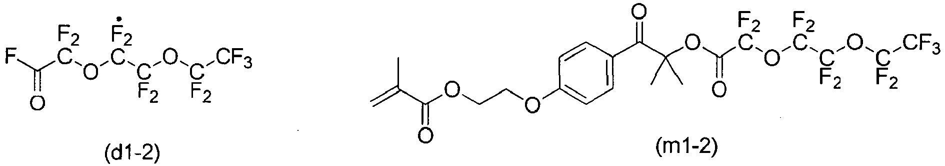

### [Example 7]

### (Preparation of compound (m1-3))

3.5 g of compound (m1-3) was obtained in the same manner as in Example 2 except that compound (b1-1) was changed to 1.2 g of methacrylic acid chloride, and compound (d1-1) was changed to 6.6 g of 2,3,3,3-tetrafluoro-2-(1,1,2,3,3,3-hexafluoro-2-(perfluoropropoxy)propoxy)propanoyl fluoride (compound (d1-3)).

Compound (m1-3) was identified by ¹H-NMR and ¹⁹F-NMR.
NMR spectra of compound (m1-3):
¹H-NMR(300.4MHz, solvent:CDCl₃, standard:TMS)δ(ppm):1.85(s, 6H), 1.95(s, 3H), 4.27(t, J=4.8, 2H), 4.52(t, J=4.8, 2H), 5.60(s, 1 H), 6.13(s, 1 H), 6.92(d, J=8.7, 2H), 7.92(d, J=8.7, 2H).
¹⁹F-NMR(282.7MHz, solvent:CDCl₃, standard:CFCl₃)δ(ppm):-145.6(m, 1 F),
-132.3(m, 1 F), -130.0(m, 2F), -84.0(m, 1 F), -82.0(m, 5F), -81.8(m, 3F), -80.5(m, 3F), -79.7(m, 1 F).

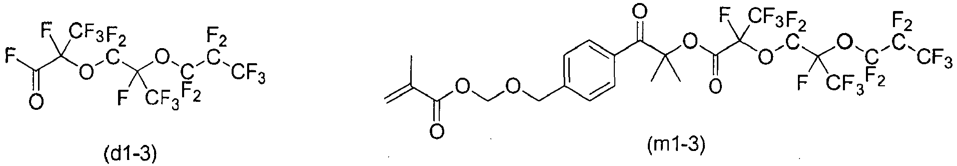

### [Example 8]

### (Preparation of liquid repellent polymer (C-2))

In 2.3 g of 2-butanone, 0.80 g of compound (m1-2) obtained in Example 6 and 0.17 g of 2-hydroxyethyl methacrylate were reacted at 50°C for 24 hours in the presence of 0.037 g of n-dodecylmercaptan and 0.005 g of 2,2'-azobis(2,4-dimethylvaleronitrile) (manufactured by Wako Pure Chemical Industries, Ltd., tradename: V-65). After the reaction mixture was cooled to room temperature (20 to 25°C), 0.18 g of 2-acryloyloxyethyl isocyanate, 0.0007 g of dibutyltin dilaurate and 0.0091 g of 2,6-di-tert-butyl-p-cresol were added, followed by reaction at 40°C for 24 hours to obtain a 2-butanone solution of liquid repellent polymer (C-2) having the following units (u1-2) and (u2-1). The obtained 2-butanone solution of liquid repellent polymer (C-2) was poured into hexane to precipitate a solid, which was vacuum-dried to obtain 0.87 g of powdery liquid repellent polymer (C-2). The fluorine content and the number average molecular weight of liquid repellent polymer (C-2) are shown in Table 1.

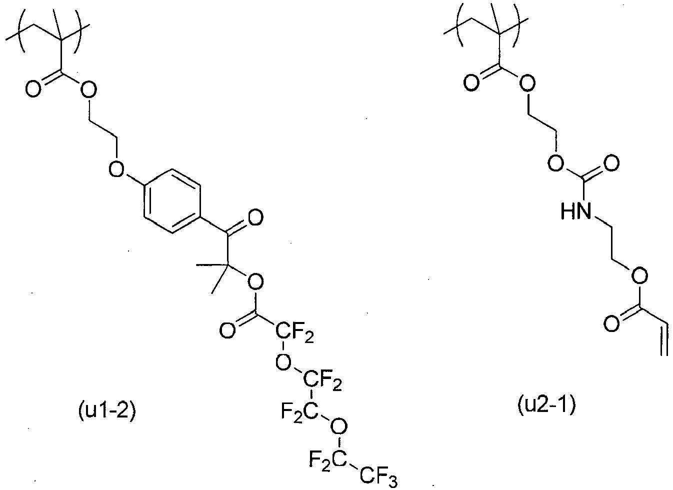

### [Examples 9 to 11]

### (Preparation of liquid repellent polymers (C-3) to (C-5))

Powdery liquid repellent polymers (C-3) to (C-5) were prepared in the same manner as in Example 8 except for the blend amounts as identified in Table 1. The fluorine contents and the number average molecular weights of the obtained liquid repellent polymers are shown in Table 1.

### [Example 12]

### (Preparation of liquid repellent polymer (C-6))

In 2.2 g of 2-butanone, 0.80 g of compound (m1-2), 0.03 g of 2-hydroxyethyl methacrylate and 0.04 g of octyl acrylate were reacted at 50°C for 24 hours in the presence of 0.031 g of n-dodecylmercaptan and 0.004 g of V-65. After the reaction mixture was cooled to room temperature (20 to 25°C), 0.09 g of 2-acryloyloxyethyl isocyanate, 0.0004 g of dibutyltin dilaurate and 0.0045 g of 2,6-di-tert-butyl-p-cresol were added, followed by reaction at 40°C for 24 hours to obtain a 2-butanone solution of liquid repellent polymer (C-6) having the following units (u1-2), (u2-1) and (u3-1). The obtained 2-butanone solution of liquid repellent polymer (C-6) was poured into hexane to precipitate a solid, which was vacuum-dried to obtain 0.72 g of powdery liquid repellent polymer (C-6). The fluorine content and the number average molecular weight of liquid repellent polymer (C-6) are shown in Table 1.

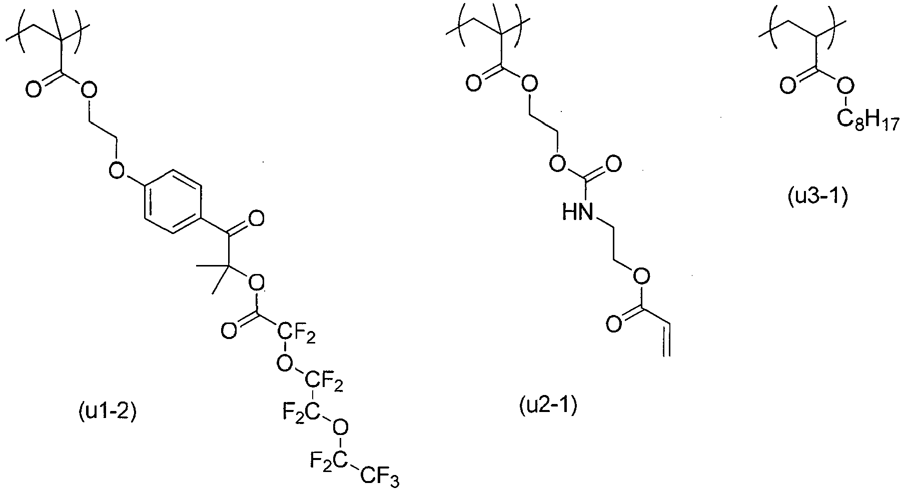

### [Examples 13 to 15]

### (Preparation of liquid repellent polymers (C-7) to (C-9))

Powdery liquid repellent polymers (C-7) to (C-9) were prepared in the same manner as in Example 12 except for the blend amounts as identified in Table 1. The fluorine contents and the number average molecular weights of the obtained liquid repellent polymers are shown in Table 1.

### [Example 16]

### (Preparation of liquid repellent polymer (C-10))

In 2.4 g of 2-butanone, 0.90 g of compound (m1-2), 0.03 g of 2-hydroxyethyl methacrylate and 0.08 g of styrene were reacted at 50°C for 24 hours in the presence of 0.035 g of n-dodecylmercaptan and 0.005 g of V-65. After the reaction mixture was cooled to room temperature (20 to 25°C), 0.03 g of 2-acryloyloxyethyl isocyanate, 0.0001 g of dibutyltin dilaurate and 0.0017 g of 2,6-di-tert-butyl-p-cresol were added, followed by reaction at 40°C for 24 hours to obtain a 2-butanone solution of liquid repellent polymer (C-10) having the following units (u1-2), (u2-1) and (u3-2). The obtained 2-butanone solution of liquid repellent polymer (C-10) was poured into hexane to precipitate a solid, which was vacuum-dried to obtain 0.44 g of powdery liquid repellent polymer (C-10). The fluorine content and the number average molecular weight of liquid repellent polymer (C-10) are shown in Table 1.

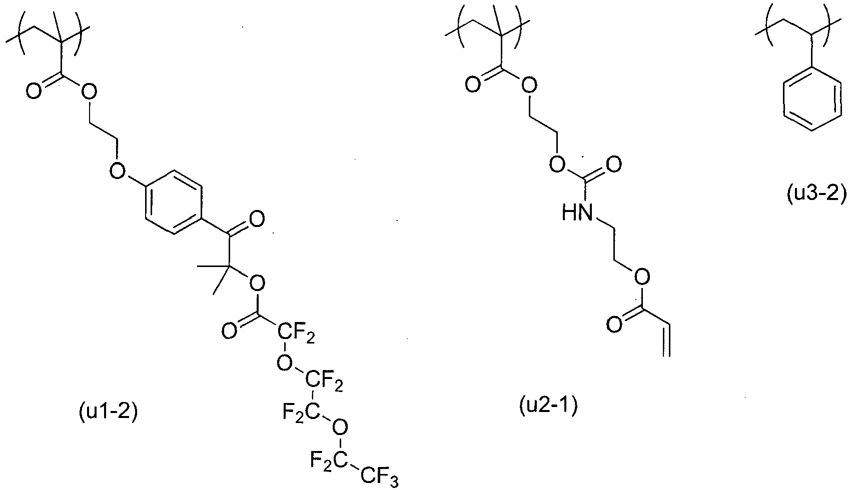

### [Example 17]

### (Preparation of liquid repellent polymer (C-11))

Powdery liquid repellent polymer (C-11) was prepared in the same manner as in Example 16 except for the blend amount as identified in Table 1. The fluorine content and the number average molecular weight of the obtained liquid repellent polymer are shown in Table 1.

### [Example 18]

### (Preparation of liquid repellent polymer (C-12))

In 2.8 g of 2-butanone, 1.00 g of compound (m1-2), 0.04 g of 2-hydroxyethyl methacrylate and 0.14 g of hexyl methacrylate were reacted at 50°C for 24 hours in the presence of 0.039 g of n-dodecylmercaptan and 0.005 g of V-65. After the reaction mixture was cooled to room temperature (20 to 25°C), 0.04 g of 2-acryloyloxyethyl isocyanate, 0.0002 g of dibutyltin dilaurate and 0.0019 g of 2,6-di-tert-butyl-p-cresol were added, followed by reaction at 40°C for 24 hours to obtain a 2-butanone solution of liquid repellent polymer (C-12) having the following units (u1-2), (u2-1) and (u3-3). The obtained 2-butanone solution of liquid repellent polymer (C-12) was poured into hexane to precipitate a solid, which was vacuum-dried to obtain 0.88 g of powdery liquid repellent polymer (C-12). The fluorine content and the number average molecular weight of liquid repellent polymer (C-12) are shown in Table 1.

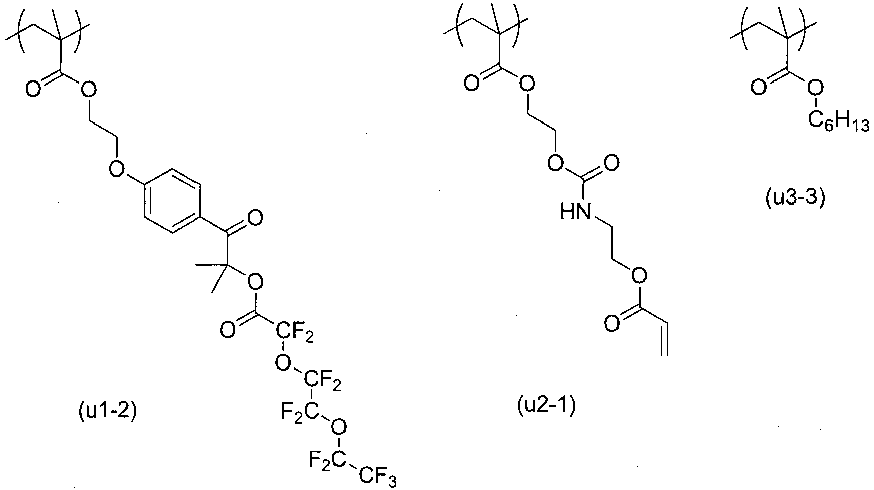

### [Example 19]

### (Preparation of liquid repellent polymer (C-13))

Powdery liquid repellent polymer (C-13) was prepared in the same manner as in Example 18 except for the blend amount as identified in Table 1. The fluorine content and the number average molecular weight of the obtained liquid repellent polymer are shown in Table 1.

### [Example 20]

### (Preparation of liquid repellent polymer (C-14))

In 2.6 g of 2-butanone, 0.90 g of compound (m1-2), 0.03 g of 2-hydroxyethyl methacrylate and 0.18 g of dodecyl methacrylate were reacted at 50°C for 24 hours in the presence of 0.035 g of n-dodecylmercaptan and 0.005 g of V-65. After the reaction mixture was cooled to room temperature (20 to 25°C), 0.03 g of 2-acryloyloxyethyl isocyanate, 0.0001 g of dibutyltin dilaurate and 0.0017 g of 2,6-di-tert-butyl-p-cresol were added, followed by reaction at 40°C for 24 hours to obtain a 2-butanone solution of liquid repellent polymer (C-14) having the following units (u1-2), (u2-1) and (u3-4). The obtained 2-butanone solution of liquid repellent polymer (C-14) was poured into hexane to precipitate a solid, which was vacuum-dried to obtain 0.72 g of powdery liquid repellent polymer (C-14). The fluorine content and the number average molecular weight of liquid repellent polymer (C-14) are shown in Table 2.

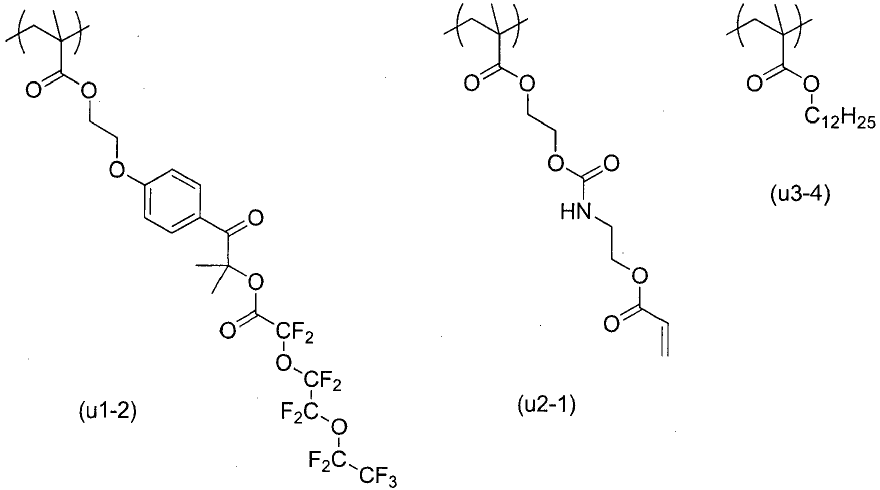

### [Example 21]

### (Preparation of liquid repellent polymer (C-15))

Powdery liquid repellent polymer (C-15) was prepared in the same manner as in Example 20 except for the blend amount as identified in Table 1. The fluorine content and the number average molecular weight of the obtained liquid repellent polymer are shown in Table 2.

### [Example 22]

### (Preparation of liquid repellent polymer (C-16))

In 2.7 g of 2-butanone, 1.00 g of compound (m1-2), 0.04 g of 2-hydroxyethyl methacrylate and 0.08 g of methyl methacrylate were reacted at 50°C for 24 hours in the presence of 0.039 g of n-dodecylmercaptan and 0.005 g of V-65. After the reaction mixture was cooled to room temperature (20 to 25°C), 0.04 g of 2-acryloyloxyethyl isocyanate, 0.0002 g of dibutyltin dilaurate and 0.0019 g of 2,6-di-tert-butyl-p-cresol were added, followed by reaction at 40°C for 24 hours to obtain a 2-butanone solution of liquid repellent polymer (C-16) having the following units (u1-2), (u2-1) and (u3-5). The obtained 2-butanone solution of liquid repellent polymer (C-16) was poured into hexane to precipitate a solid, which was vacuum-dried to obtain 0.72 g of powdery liquid repellent polymer (C-16). The fluorine content and the number average molecular weight of liquid repellent polymer (C-16) are shown in Table 2.

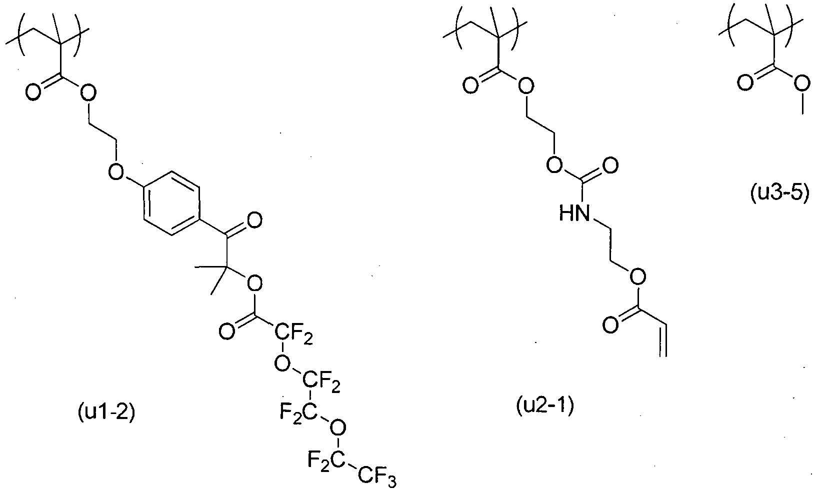

### [Example 23]

### (Preparation of liquid repellent polymer (C-17))

Powdery liquid repellent polymer (C-17) was prepared in the same manner as in Example 22 except for the blend amount as identified in Table 1. The fluorine content and the number average molecular weight of the obtained liquid repellent polymer are shown in Table 2.

### [Example 24]

### (Preparation of liquid repellent polymer (C-18))

In 2.7 g of 2-butanone, 1.00 g of compound (m1-2), 0.04 g of 2-hydroxyethyl methacrylate and 0.11 g of butyl methacrylate were reacted at 50°C for 24 hours in the presence of 0.039 g of n-dodecylmercaptan and 0.005 g of V-65. After the reaction mixture was cooled to room temperature (20 to 25°C), 0.04 g of 2-acryloyloxyethyl isocyanate, 0.0002 g of dibutyltin dilaurate and 0.0019 g of 2,6-di-tert-butyl-p-cresol were added, followed by reaction at 40°C for 24 hours to obtain a 2-butanone solution of liquid repellent polymer (C-18) having the following units (u1-2), (u2-1) and (u3-6). The obtained 2-butanone solution of liquid repellent polymer (C-18) was poured into hexane to precipitate a solid, which was vacuum-dried to obtain 0.91 g of powdery liquid repellent polymer (C-18). The fluorine content and the number average molecular weight of liquid repellent polymer (C-18) are shown in Table 2.

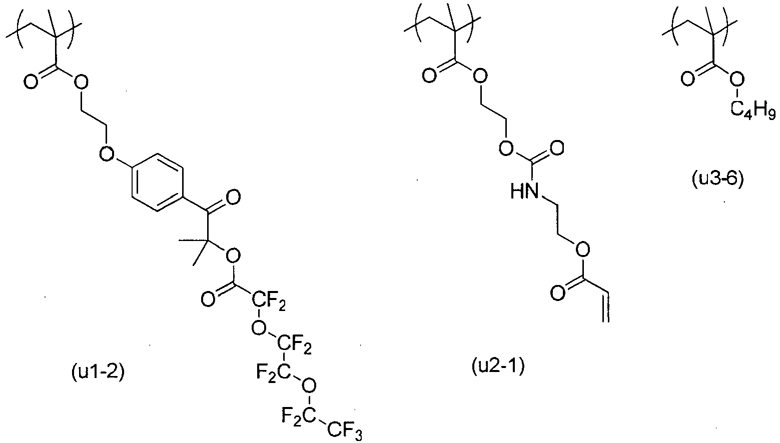

### [Example 25]

### (Preparation of liquid repellent polymer (C-19))

In 2.5 g of 2-butanone, 0.80 g of compound (m1-2), 0.03 g of 2-hydroxyethyl methacrylate and 0.21 g of RUVA-93 (tradename, manufactured by Otsuka Chemical Co., Ltd.) were reacted at 50°C for 24 hours in the presence of 0.031 g of n-dodecylmercaptan and 0.004 g of V-65. After the reaction mixture was cooled to room temperature (20 to 25°C), 0.03 g of 2-acryloyloxyethyl isocyanate, 0.0001 g of dibutyltin dilaurate and 0.0015 g of 2,6-di-tert-butyl-p-cresol were added, followed by reaction at 40°C for 24 hours to obtain a 2-butanone solution of liquid repellent polymer (C-19) having the following units (u1-2), (u2-1) and (u3-7). The obtained 2-butanone solution of liquid repellent polymer (C-19) was poured into hexane to precipitate a solid, which was vacuum-dried to obtain 0.66 g of powdery liquid repellent polymer (C-19). The fluorine content and the number average molecular weight of liquid repellent polymer (C-19) are shown in Table 2.

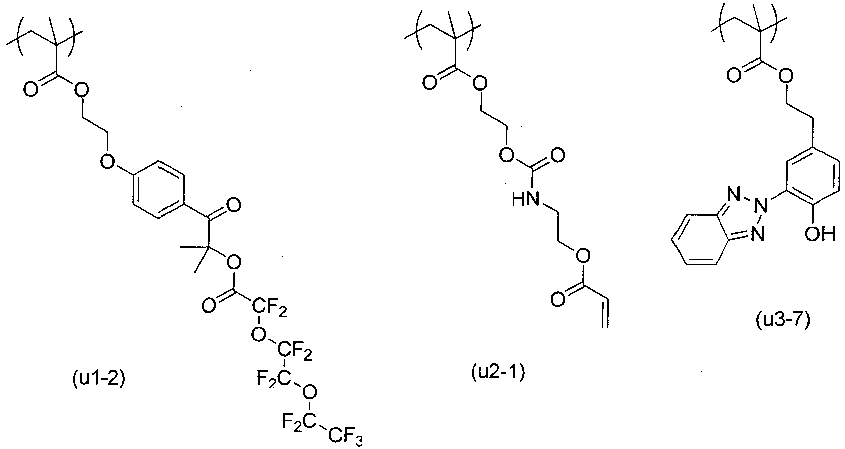

### [Example 26]

### (Preparation of liquid repellent polymer (C-20))

In 2.2 g of 2-butanone, 0.80 g of compound (m1-3) obtained in Example 7 and 0.14 g of 2-hydroxyethyl methacrylate were reacted at 50°C for 24 hours in the presence of 0.030 g of n-dodecylmercaptan and 0.004 g of V-65. After the reaction mixture was cooled to room temperature (20 to 25°C), 0.15 g of 2-acryloyloxyethyl isocyanate, 0.0006 g of dibutyltin dilaurate and 0.0073 g of 2,6-di-tert-butyl-p-cresol were added, followed by reaction at 40°C for 24 hours to obtain a 2-butanone solution of liquid repellent polymer (C-20) having the following units (u1-3) and (u2-1). The obtained 2-butanone solution of liquid repellent polymer (C-20) was poured into hexane to precipitate a solid, which was vacuum-dried to obtain 0.76 g of powdery liquid repellent polymer (C-20). The fluorine content and the number average molecular weight of liquid repellent polymer (C-20) are shown in Table 2.

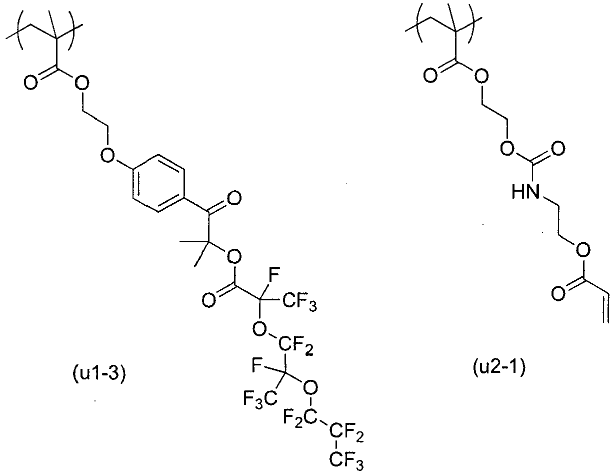

### [Examples 27 to 29]

### (Preparation of liquid repellent polymers (C-21) to (C-23))

Powdery liquid repellent polymers (C-21) to (C-23) were prepared in the same manner as in Example 26 except for the blend amounts as identified in Table 1. The fluorine contents and the number average molecular weights of the obtained liquid repellent polymers are shown in Table 2.

### [Example 30]

### (Preparation of liquid repellent polymer (C-24))

In 2.6 g of 2-butanone, 0.80 g of compound (m1-3), 0.03 g of 2-hydroxyethyl methacrylate and 0.24 g of octyl acrylate were reacted at 50°C for 24 hours in the presence of 0.037 g of n-dodecylmercaptan and 0.005 g of V-65. After the reaction mixture was cooled to room temperature (20 to 25°C), 0.04 g of 2-acryloyloxyethyl isocyanate, 0.0001 g of dibutyltin dilaurate and 0.0018 g of 2,6-di-tert-butyl-p-cresol were added, followed by reaction at 40°C for 24 hours to obtain a 2-butanone solution of liquid repellent polymer (C-24) having the following units (u1-3), (u2-1) and (u3-1). The obtained 2-butanone solution of liquid repellent polymer (C-24) was poured into hexane to precipitate a solid, which was vacuum-dried to obtain 0.57 g of powdery liquid repellent polymer (C-24). The fluorine content and the number average molecular weight of liquid repellent polymer (C-24) are shown in Table 2.

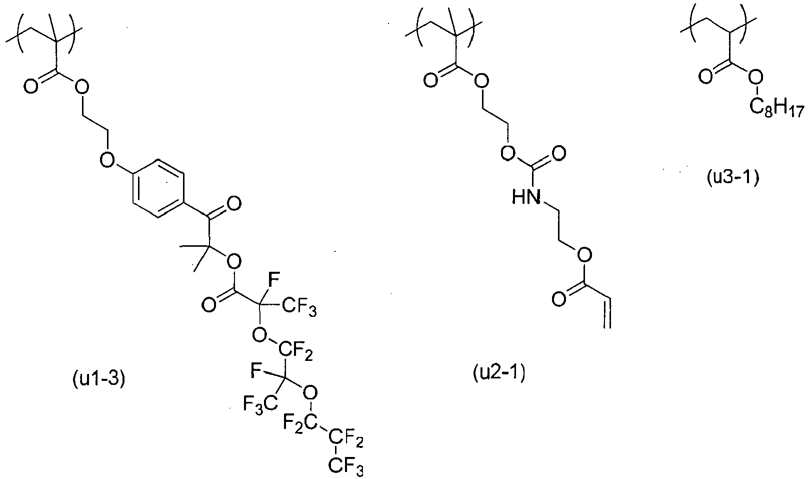

### [Example 31]

### (Preparation of liquid repellent polymer (C-25))

Powdery liquid repellent polymer (C-25) was prepared in the same manner as in Example 24 except for the blend amount as identified in Table 1. The fluorine content and the number average molecular weight of the obtained liquid repellent polymer are shown in Table 2.

**[Table 1]**

| | | Ex.8 | Ex.9 | Ex.10 | Ex.11 | Ex.12 | Ex.13 | Ex.14 | Ex.15 | Ex.16 | Ex.17 | Ex.18 | Ex.19 |
|---|---|---|---|---|---|---|---|---|---|---|---|---|---|
| Blend amount (g) | 2-Butanone | 2.3 | 2.2 | 2.6 | 2.5 | 2.2 | 2.5 | 2.7 | 2.8 | 2.4 | 2.4 | 2.8 | 2.7 |
| | Compound (m1-2) | 0.80 | 0.80 | 1.00 | 1.00 | 0.80 | 0.90 | 0.80 | 0.70 | 0.90 | 0.80 | 1.00 | 0.80 |
| | Compound (m1-3) | - | - | - | - | - | - | - | - | - | - | - | - |
| | 2-Hydroxyethyl methacrylate | 0.17 | 0.11 | 0.09 | 0.05 | 0.03 | 0.03 | 0.04 | 0.05 | 0.03 | 0.04 | 0.04 | 0.04 |
| | Octyl acrylate | - | - | - | - | 0.04 | 0.13 | 0.30 | 0.42 | - | - | - | - |
| | Styrene | - | - | - | - | - | - | - | - | 0.08 | 0.17 | - | - |
| | Hexyl methacrylate | - | - | - | - | - | - | - | - | - | - | 0.14 | 0.27 |
| | Dodecyl methacrylate | - | - | - | - | - | - | - | - | - | - | - | - |
| | Methyl methacrylate | - | - | - | - | - | - | - | - | - | - | - | - |
| | Butyl methacrylate | - | - | - | - | - | - | - | - | - | - | - | - |
| | RUVA-93 | - | - | - | - | - | - | - | - | - | - | - | - |
| | n-Dodecylmercaptan | 0.037 | 0.031 | 0.033 | 0.029 | 0.031 | 0.035 | 0.046 | 0.034 | 0.035 | 0.046 | 0.039 | 0.046 |
| | V-65 | 0.005 | 0.004 | 0.005 | 0.004 | 0.004 | 0.005 | 0.006 | 0.008 | 0.005 | 0.006 | 0.005 | 0.006 |
| | 2-Acryloyloxyethyl isocyanate | 0.18 | 0.12 | 0.10 | 0.06 | 0.09 | 0.03 | 0.05 | 0.05 | 0.03 | 0.05 | 0.04 | 0.05 |
| | Dibutyltin dilaurate | 0.0007 | 0.0005 | 0.0004 | 0.0002 | 0.0004 | 0.0001 | 0.0002 | 0.0002 | 0.0001 | 0.0002 | 0.0002 | 0.0002 |
| | 2,6-di-tert-Butyl-p-cresol | 0.0091 | 0.0061 | 0.0049 | 0.0028 | 0.0045 | 0.0017 | 0.0023 | 0.0027 | 0.0017 | 0.0023 | 0.0019 | 0.0023 |
| Liquid repellent polymer | Kind | (C-2) | (C-3) | (C-4) | (C-5) | (C-6) | (C-7) | (C-8) | (C-9) | (C-10) | (C-11) | (C-12) | (C-13) |
| | Units contained | (u1-2) | (u1-2) | (u1-2) | (u1-2) | (u1-2) | (u1-2) | (u1-2) | (u1-2) | (u1-2) | (u1-2) | (u1-2) | (u1-2) |
| | | (u2-1) | (u2-1) | (u2-1) | (u2-1) | (u2-1) | (u2-1) | (u2-1) | (u2-1) | (u2-1) | (u2-1) | (u2-1) | (u2-1 ) |
| | | - | - | - | - | (u3-1) | (u3-1) | (u3-1) | (u3-1) | (u3-2) | (u3-2) | (u3-3) | (u3-3) |
| | Proportion of units (u1) (mass%) | 57.5 | 67.0 | 75.9 | 84.4 | 68.6 | 78.6 | 64.2 | 72.1 | 54.2 | 43.4 | 73.1 | 55.7 |
| | Proportion of units (u2) (mass%) | 42.5 | 33.0 | 24.1 | 15.6 | 25.4 | 9.7 | 11.9 | 8.9 | 10.0 | 10.7 | 9.0 | 10.3 |
| | Proportion of units (u3) (mass%) | - | - | - | - | 6.1 | 11.8 | 24.0 | 19.1 | 35.8 | 45.9 | 17.9 | 34.0 |
| | Amount (g) obtained | 0.87 | 0.81 | 0.87 | 0.82 | 0.72 | 0.78 | 0.86 | 0.53 | 0.44 | 0.40 | 0.88 | 0.74 |
| Fluorine content (mass%) | | 34.5 | 40.2 | 45.6 | 50.7 | 41.2 | 43.3 | 32.5 | 26.0 | 47.2 | 38.5 | 43.9 | 33.4 |
| Number average molecular weight (Mn) | | 17,000 | 10,000 | 13,000 | 10,000 | 14,000 | 11,000 | 8,000 | 8,000 | 10,000 | 7,000 | 10,000 | 9,000 |

**[Table 2]**

| | | Ex.20 | Ex.21 | Ex.22 | Ex.23 | Ex.24 | Ex.25 | Ex.26 | Ex.27 | Ex.28 | Ex.29 | Ex.30 | Ex.31 |
|---|---|---|---|---|---|---|---|---|---|---|---|---|---|
| Blend amount (g) | 2-Butanone | 2.6 | 2.6 | 2.7 | 2.7 | 2.7 | 2.5 | 2.2 | 2.1 | 2.0 | 2.0 | 2.6 | 2.6 |
| | Compound (m1-2) | 0.90 | 0.70 | 1.00 | 0.90 | 1.00 | 0.80 | - | - | - | - | - | - |
| | Compound (m1-3) | - | - | - | - | - | - | 0.80 | 0.80 | 0.80 | 0.80 | 0.80 | 0.70 |
| | 2-Hydroxyethyl methacrylate | 0.03 | 0.04 | 0.04 | 0.05 | 0.04 | 0.03 | 0.14 | 0.09 | 0.06 | 0.03 | 0.03 | 0.04 |
| | Octyl acrylate | - | - | - | - | - | - | - | -- | | - | 0.24 | 0.33 |
| | Styrene | - | - | - | - | - | - | - | - | - | - | - | - |
| | Hexyl methacrylate | - | - | - | - | - | - | - | - | - | - | - | - |
| | Dodecyl methacrylate | 0.18 | 0.36 | - | - | - | - | - | - | - | - | - | - |
| | Methyl methacrylate | - | - | 0.08 | 0.18 | - | - | - | - | - | - | - | - |
| | Butyl methacrylate | - | - | - | - | 0.11 | - | - | - | - | - | - | - |
| | RUVA-93 | - | - | - | - | - | 0.21 | - | - | - | - | - | - |
| | n- Dodecylmercaptan | 0.035 | 0.040 | 0.039 | 0.052 | 0.039 | 0.031- | 0.030 | 0.025 | 0.021 | 0.019 | 0.037 | 0.043 |
| | V-65 | 0.005 | 0.006 | 0.005 | 0.007 | 0.005 | 0.004 | 0.004 | 0.003 | 0.003 | 0.003 | 0.005 | 0.006 |
| | 2-Acryloyloxyethyl isocyanate | 0.03 | 0.04 | 0.04 | 0.05 | 0.04 | 0.03 | 0.15 | 0.10 | 0.06 | 0.04 | 0.04 | 0.04 |
| | Dibutyltin dilaurate | 0.0001 | 0.0002 | 0.0002 | 0.0002 | 0.0002 | 0.0001 | 0.0006 | 0.0004 | 0.0003 | 0.0001 | 0.0001 | 0.0002 |
| | 2,6-di-tert-Butyl-p-cresol | 0.0017 | 0.0020 | 0.0019 | 0.0026 | 0.0019 | 0.0015 | 0.0073 | 0.0049 | 0.0031 | 0.0018 | 0.0018 | 0.0021 |
| Liquid repellent polymer | Kind | (C-14) | (C-15) | (C-16) | (C-17) | (C-18) | (C-19) | (C-20) | (C-21) | (C-22) | (C-23) | (C-24) | (C-25) |
| | Units contained | (u1-2) | (u1-2) | (u1-2) | (u1-2) | (u1-2) | (u1-2) | (u1-3) | (u1-3) | (u1-3) | (u1-3) | (u1-3) | (u1-3) |
| | | (u2-1) | (u2-1) | (u2-1) | (u2-1) | (u2-1) | (u2-1) | (u2-1) | (u2-1) | (u2-1) | (u2-1) | (u2-1) | (u2-1) |
| | | (u3-4) | (u3-4) | (u3-5) | (u3-5) | (u3-6) | (u3-7) | - | - | - | - | (u3-1) | (u3-1) |
| | Proportion of units (u1) (mass%) | 67.2 | 47.7 | 78.9 | 64.8 | 75.3 | 63.0 | 65.9 | 74.4 | 81.9 | 88.6 | 62.8 | 52.3 |
| | Proportion of units (u2) (mass%) | 8.3 | 8.8 | 9.7 | 12.0 | 9.3 | 7.8 | 34.1 | 25.6 | 18.1 | 11.4 | 8.1 | 9.0 |
| | Proportion of units (u3) (mass%) | 24.6 | 43.5 | 11.4 | 23.3 | 15.4 | 29.3 | - | - | - | - | 29.1 | 38.7 |
| | Amount (g) obtained | 0.72 | 0.66 | 0.72 | 0.86 | 0.91 | 0.66 | 0.76 | 0.62 | 0.49 | 0.50 | 0.57 | 0.72 |
| Fluorine content (mass%) | | 40.3 | 28.6 | 47.4 | 38.9 | 45.2 | 37.8 | 42.8 | 48.2 | 53.1 | 57.4 | 40.7 | 33.9 |
| Number average molecular weight (Mn) | | 12,000 | 9,000 | 11,000 | 8,000 | 11,000 | 15,000 | 12,000 | 11,000 | 11,000 | 9,000 | 9,000 | 9,000 |

### INDUSTRIAL APPLICABILITY

With the curable composition of the present invention, formation of an article having a pattern of a liquid-philic region and a liquid repellent region on the surface of an obtainable cured film is easy, and is useful for an electrical insulation film, a chemical or physical protective film, a non-adhesive film, etc. in various electronic devices (such as a semiconductor device). Specifically, it is useful for application to an interlayer insulation film for a flexible device, a protective film for a flexible device, a gate insulation film for an organic thin-film transistor, a gate insulation film for an oxide thin-film transistor, a capacitor insulation film, a gate insulation film of a memory transistor, a passivation of a semiconductor, a protective film of a semiconductor device, an interlayer insulation film of multilayer interconnection for high density mounting, an insulating layer of an organic electroluminescence device, an insulation film for re-wiring, a cover coating of a flexible copper-clad plate, a solder resist film, a liquid crystal alignment film, a protective film for a color filter, a resin post for e.g. a semiconductor device, and partition walls for e.g. a color filter, etc.

### REFERENCE SYMBOLS

- 10:: Organic thin-film transistor
- 12:: Substrate
- 14:: Gate electrode
- 16:: Gate insulation film
- 16a:: Liquid-philic region
- 16b:: Liquid repellent region
- 16c:: Internal region
- 18:: Source electrode
- 20:: Drain electrode
- 22:: Organic semiconductor layer

## Claims

1. A liquid repellent compound represented by the following formula (m1 ): 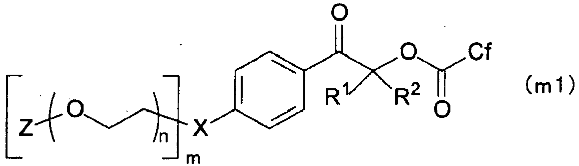 wherein Cf is a C₁₋₂₀ fluoroalkyl group or a C₁₋₂₀ fluoroalkyl group having an etheric oxygen atom between carbon atoms,
each of R¹ and R² which are independent of each other, is a hydrogen atom, a C₁₋₆ alkyl group or a phenyl group,
X is an oxygen atom, a sulfur atom, a nitrogen atom or NH,
n is an integer of from 0 to 4,
m is 1 when X is an oxygen atom, a sulfur atom or NH, or 2 when X is a nitrogen atom,
Z is R⁴R⁵C=CR³-CO-, and
each of R³, R⁴ and R⁵ which are independent of one another, is a hydrogen atom or a methyl group.

2. A liquid repellent polymer having units (u1) where the unit (u1) is a unit derived from the compound (m1) formed by polymerization of the compound (m1) as defined in Claim 1,

3. The liquid repellent polymer according to Claim 2, which further has units (u2) having a crosslinkable functional group and having no Cf group.

4. A curable composition comprising the liquid repellent polymer as defined in Claim 2 or 3.

5. The curable composition according to Claim 4, which further contains a radical polymerization initiator (D).

6. The curable composition according to Claim 4 or 5, which further contains a fluorinated polyarylene prepolymer (A) having a crosslinkable functional group.

7. The curable composition according to Claim 6, which further contains a compound (B) having a number average molecular weight of from 140 to 5,000, having a crosslinkable functional group and having no fluorine atoms.

8. The curable composition according to any one of Claims 4 to 7, wherein the fluorinated polyarylene prepolymer (A) is a prepolymer having a crosslinkable functional group and an ether bond, obtained by subjecting either one or both of a compound (x1) having a crosslinkable functional group and a phenolic hydroxy group and a compound (x2) having a crosslinkable functional group and a fluorinated aromatic ring, a compound (y) represented by the following formula (y): 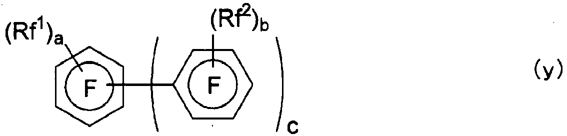 wherein a is an integer of from 0 to 3, b is an integer of from 0 to 3, c is an integer of from 0 to 3, each of Rf¹ and Rf² which are independent of each other, is a fluoroalkyl group having at most 8 carbon atoms, and F in the aromatic ring represents that hydrogen atoms of the aromatic ring are all substituted by fluorine atoms; and a compound (z) having at least three phenolic hydroxy groups, to a condensation reaction in the presence of a hydrogen halide-removing agent.

9. A coating composition comprising the curable composition as defined in any one of Claims 4 to 8 and a solvent (E).

10. An article comprising a substrate, and a cured film obtained by curing the curable composition as defined in any one of Claims 4 to 8 on the surface of the substrate.

11. An article having a pattern of a liquid-philic region and a liquid repellent region on the surface of a cured film, wherein the liquid repellent region comprises a cured film obtained by curing the curable composition as defined in any one of Claims 4 to 8.

12. A process for producing an article having a pattern of a liquid-philic region and a liquid repellent region on the surface of a cured film, which comprises the following steps (I) and (II):
(I) a step of applying the coating composition as defined in Claim 9 on the surface of a substrate, and removing the solvent (E), followed by heating or light irradiation to form a cured film, and
(II) a step of partially irradiating the surface of the cured film with ultraviolet light to obtain an article having a pattern of a liquid-philic region and a liquid repellent region on the surface of the cured film.

13. The process for producing an article according to Claim 12, which further has the following step (III) after the step (II):
(III) a step of selectively depositing at least one member selected from the group consisting of a composition for an electrode, a composition for a semiconductor layer, a composition for a conductor layer, a composition for a transistor material and a composition for a resin layer to the surface of the liquid-philic region to form at least one member selected from the group consisting of an electrode, a semiconductor layer, a conductor layer, a transistor material and a resin layer.

14. The article according to Claim 10, which further has at least one member selected from the group consisting of an electrode, a semiconductor layer, a conductor layer, a transistor material and a resin layer, formed on the surface of the liquid-philic region.

15. The article according to Claim 14, which is a thin-film transistor.

## Patentansprüche

1. Flüssigkeitsabweisende Verbindung, dargestellt durch die folgende Formel (m1): 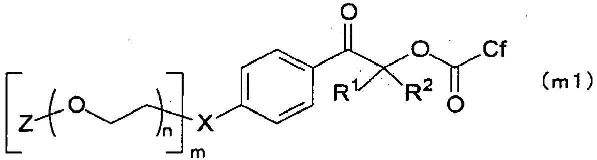 worin Cf eine C₁₋₂₀ Fluoralkylgruppe oder eine C₁₋₂₀ Fluoralkylgruppe mit einem Ethersauerstoffatom zwischen Kohlenstoffatomen ist,
wobei R¹ und R², welche unabhängig voneinander sind, jeweils ein Wasserstoff, eine C₁₋₆ Alkylgruppe oder eine Phenylgruppe sind,
X ein Sauerstoffatom, ein Schwefelatom, ein Stickstoffatom oder NH ist,
n eine ganze Zahl von 0 bis 4 ist,
m 1 ist, wenn X ein Sauerstoffatom, ein Schwefelatom oder NH ist, oder 2 ist, wenn X ein Stickstoffatom ist,
Z R⁴R⁵C=CR³-CO- ist, und
R³, R⁴ und R⁵, welche unabhängig voneinander sind, jeweils ein Wasserstoffatom oder eine Methylgruppe sind.

2. Flüssigkeitsabweisendes Polymer mit Einheiten (u1), wobei die Einheit (u1) eine Einheit ist, abgeleitet von der Verbindung (m1), gebildet durch Polymerisation der Verbindung (m1), wie in Anspruch 1 definiert.

3. Flüssigkeitsabweisendes Polymer gemäß Anspruch 2, welches weiter Einheiten (u2) mit einer vernetzbaren funktionalen Gruppe und mit keiner Cf Gruppe aufweist.

4. Härtbare Zusammensetzung, umfassend das flüssigkeitsabweisende Polymer, wie in Anspruch 2 oder 3 definiert.

5. Härtbare Zusammensetzung gemäß Anspruch 4, welche weiter einen radikaiischen Polymerisationsinitiator (D) enthält.

6. Härtbare Zusammensetzung gemäß Anspruch 4 oder 5, welche weiter ein fluoriertes Polyarylenpräpolymer (A) mit einer vernetzbaren funktionalen Gruppe enthält.

7. Härtbare Zusammensetzung gemäß Anspruch 6, welche weiter eine Verbindung (B) mit einem zahlenmittleren Molekulargewicht von 140 bis 5000 mit einer vernetzbaren funktionalen Gruppe und mit keinen Fluoratomen enthält.

8. Härtbare Zusammensetzung gemäß einem der Ansprüche 4 bis 7, wobei das fluorierte Polyarylenpräpolymer (A) ein Präpolymer mit einer vernetzbaren funktionalen Gruppe und einer Etherbindung ist, erhalten durch Unterwerfen von entweder einem oder beiden einer Verbindung (x1) mit einer vernetzbaren funktionalen Gruppe und einer phenolischen Hydroxygruppe und einer Verbindung (x2) mit einer vernetzbaren funktionellen Gruppe und einem fluorierten aromatischen Ring einer Verbindung (y), dargestellt durch die folgende Formel (y): 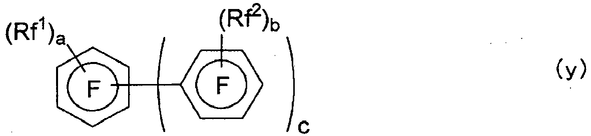 (worin a eine ganze Zahl von 0 bis 3 ist, b eine ganze Zahl von 0 bis 3 ist, c eine ganze Zahl von 0 bis 3 ist, Rf¹ und Rf², welche unabhängig voneinander sind, jeweils eine Fluoralkylgruppe mit höchstens acht Kohlenstoffatomen sind, und F in dem aromatischen Ring darstellt, dass Wasserstoffatome des aromatischen Rings sämtlich durch Fluoratome substituiert sind,
und einer Verbindung (z) mit mindestens drei phenolischen Hydroxygruppen einer Kondensationsreaktion in der Gegenwart eines Wasserstoffhalogenidentfernenden Mittels.

9. Beschichtungszusammensetzung, umfassend die härtbare Zusammensetzung, wie in einem der Ansprüche 4 bis 8 definiert, und ein Lösungsmittel (E).

10. Gegenstand, umfassend ein Substrat und einen gehärteten Film, erhalten durch Härten der härtbaren Zusammensetzung, wie in einem der Ansprüche 4 bis 8 definiert, auf der Oberfläche des Substrats.

11. Gegenstand mit einem Muster eines Flüssigkeits-philen Bereichs und eines flüssigkeitsabweisenden Bereichs auf der Oberfläche eines gehärteten Films, wobei der flüssigkeitsabweisende Bereich einen gehärteten Film, erhalten durch Härten der härtbaren Zusammensetzung, wie in einem der Ansprüche 4 bis 8 definiert, umfaßt.

12. Verfahren zur Herstellung eines Gegenstands mit einem Muster eines Flüssigkeits-philen Bereichs und eines flüssigkeitsabweisenden Bereichs auf der Oberfläche eines gehärteten Films, welches die folgenden Schritte (I) und (II) umfaßt:
(I) einen Schritt des Aufbringens der Beschichtungszusammensetzung, wie in Anspruch 9 definiert, auf die Oberfläche eines Substrats und des Entfernens des Lösungsmittels (E), gefolgt durch Erwärmen oder Lichtbestrahlung unter Bildung eines gehärteten Films, und
(II) einen Schritt des teilweisen Bestrahlens der Oberfläche des gehärteten Films mit ultraviolettem Licht unter Erhalten eines Gegenstands mit einem Muster eines Flüssigkeits-philen Bereichs und eines flüssigkeitsabweisenden Bereichs auf der Oberfläche des gehärteten Films.

13. Verfahren zur Herstellung eines Gegenstands gemäß Anspruch 12, welches weiter den folgenden Schritt (III) nach dem Schritt (II) aufweist:
(III) einen Schritt des selektiven Aufbringens von mindestens einem Mitglied, ausgewählt aus der Gruppe, bestehend aus einer Zusammensetzung für eine Elektrode, einer Zusammensetzung für eine Halbleiterschicht, einer Zusammensetzung für eine Leiterschicht, einer Zusammensetzung für ein Transistormaterial und einer Zusammensetzung für eine Harzschicht, auf der Oberfläche des Flüssigkeits-philen Bereichs unter Bildung von mindestens einem Mitglied, ausgewählt aus der Gruppe, bestehend aus einer Elektrode, einer Halbleiterschicht, einer Leiterschicht, einem Transistormaterial und einer Harzschicht.

14. Gegenstand gemäß Anspruch 10, welcher weiter mindestens ein Element, ausgewählt aus der Gruppe, bestehend aus einer Elektrode, einer Halbleiterschicht, einer Leiterschicht, einem Transistormaterial und einer Harzschicht, gebildet auf der Oberfläche des Flüssigkeits-philen Bereichs, aufweist.

15. Gegenstand gemäß Anspruch 14, welcher ein Dünnfilmtransistor ist.

## Revendications

1. Composé repoussant les liquides, représenté par la formule (m1) suivante : 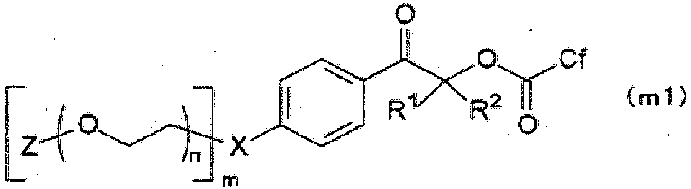 dans laquelle Cf est un groupe fluoroalkyle en C₁ à C₂₀ ou un groupe fluoroalkyle en C₁ à C₂₀ contenant un atome d'oxygène éthérique entre les atomes de carbone,
chacun parmi R¹ et R², qui sont indépendants l'un de l'autre, est un atome d'hydrogène, un groupe alkyle en C₁ à C₆ ou un groupe phényle,
X est un atome d'oxygène, un atome de soufre, un atome d'azote ou NH,
n est
un nombre entier allant de 0 à 4,
m est 1 quand X est un atome d'oxygène, un atome de soufre ou NH, ou 2 quand X est un atome d'azote,
Z est R⁴R⁵C=CR³-CO-, et
chacun parmi R³, R⁴ et R^{S}, qui sont indépendants les uns des autres, est un atome d'hydrogène ou un groupe méthyle.

2. Polymère repoussant les liquides contenant des motifs (u1), où le motif (u1) est un motif dérivé du composé (m1) formé par la polymérisation du composé (m1) tel que défini dans la revendication 1.

3. Polymère repoussant les liquides selon la revendication 2, qui contient en outre des motifs (u2) contenant un groupe fonctionnel réticulable et ne contenant aucun groupe Cf.

4. Composition durcissable comprenant le polymère repoussant les liquides tel que défini dans la revendication 2 ou 3.

5. Composition durcissable selon la revendication 4, contenant en outre un initiateur de polymérisation radicalaire (D).

6. Composition durcissable selon la revendication 4 ou 5, contenant en outre un prépolymère de polyarylène fluoré (A) contenant un groupe fonctionnel réticulable.

7. Composition durcissable selon la revendication 6, contenant en outre un composé (B) possédant un poids moléculaire moyen en nombre de 140 à 5000, contenant un groupe fonctionnel réticulable et ne contenant aucun atome de fluor.

8. Composition durcissable selon l'une quelconque des revendications 4 à 7, dans laquelle le prépolymère de polyarylène fluoré (A) est un prépolymère contenant un groupe fonctionnel réticulable et une liaison éther, obtenu en soumettant un ou deux parmi un composé (x1) contenant un groupe fonctionnel réticulable et un groupe hydroxy phénolique et un composé (x2) contenant un groupe fonctionnel réticulable et un cycle aromatique fluoré, un composé (y) représenté par la formule (y) suivante : 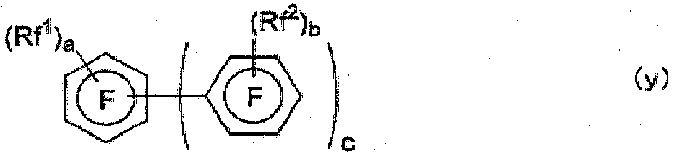 dans laquelle a est un nombre entier allant de 0 à 3, b est un nombre entier allant de 0 à 3, c est un nombre entier allant de 0 à 3, chacun parmi Rf¹ et Rf², qui sont indépendants l'un de l'autre, est un groupe fluoroalkyle contenant au plus 8 atomes de carbone, et F dans le cycle aromatique indique que les atomes d'hydrogène du cycle aromatique sont tous remplacés par des atomes de fluor ;
et un
composé (z) contenant au moins trois groupes hydroxy phénoliques, à une réaction de condensation en présence d'un agent d'élimination d'halogénure d'hydrogène.

9. Composition de revêtement comprenant la composition durcissable telle que définie dans l'une quelconque des revendications 4 à 8 et un solvant (E).

10. Article comprenant un substrat et un film durci obtenu par le durcissement de la composition durcissable telle que définie dans l'une quelconque des revendications 4 à 8 sur la surface du substrat.

11. Article comportant un motif d'une région liquidophile et d'une région repoussant les liquides sur la surface d'un film durci, dans lequel la région repoussant les liquides comprend un film durci obtenu par le durcissement de la composition durcissable telle que définie dans l'une quelconque des revendications 4 à 8.

12. Procédé de production d'un article comportant un motif d'une région liquidophile et d'une région repoussant les liquides sur la surface d'un film durci, qui comprend les étapes (I) et (II) suivantes :
(I) une étape d'application de la composition de revêtement telle que définie dans la revendication 9 sur la surface d'un substrat, et l'élimination du solvant (E), ce qui est suivi d'un chauffage ou de l'irradiation d'une lumière pour former un film durci, et
(II) une étape d'irradiation partielle de la surface du film durci avec une lumière ultraviolette pour obtenir un article comportant un motif d'une région liquidophile et d'une région repoussant les liquides sur la surface du film durci.

13. Procédé de production d'un article selon la revendication 12, qui comprend en outre l'étape (III) suivante après l'étape (II) :
(III) une étape de dépôt sélectif d'au moins un élément choisi dans le groupe constitué par une composition pour électrode, une composition pour couche de semi-conducteur, une composition pour couche de conducteur, une composition pour matériau de transistor et une composition pour couche de résine sur la surface de la région liquidophile pour former au moins un élément choisi dans le groupe constitué par une électrode, une couche de semi-conducteur, une couche de conducteur, un matériau de transistor et une couche de résine.

14. Article selon la revendication 10, qui comporte en outre au moins un élément choisi dans le groupe constitué par une électrode, une couche de semi-conducteur, une couche de conducteur, un matériau de transistor et une couche de résine, formé sur la surface de la région liquidophile.

15. Article selon la revendication 14, qui est un transistor à couche mince.
